# EUROPÄISCHE PATENTSCHRIFT

(11) **EP 3 275 403 B1**
(45) Veröffentlichungstag und Bekanntmachung des Hinweises auf die Patenterteilung: **30.12.2020**
(21) Anmeldenummer: 17183303.1
(22) Anmeldetag: 26.07.2017
(51) Int. Cl.: A61F 2/08

(54) **BEFESTIGUNGSEINHEIT SOWIE HANDHABUNGSVORRICHTUNG ZUR FADENLOSEN BEFESTIGUNG VON GEWEBE**
FIXING UNIT AND HANDLING DEVICE FOR THREAD-FREE SECURING OF TISSUE
UNITÉ DE FIXATION ET DISPOSITIF DE MANIPULATION DE FIXATION SANS FIL DE TISSU

(30) Priorität: 27.07.2016 DE 102016113797
(43) Veröffentlichungstag der Anmeldung: 31.01.2018
(73) Patentinhaber: Karl Storz SE & Co. KG, 78532 Tuttlingen (DE)
(72) Erfinder: Schanz, Steffen, 78628 Rottweil (DE)
(74) Vertreter: Witte, Weller & Partner Patentanwälte mbB

(56) Entgegenhaltungen:
- DE-T2- 60 217 981
- DE-U1- 29 922 088
- US-A- 5 013 316
- US-A- 6 056 751
- US-A- 6 162 234

## Beschreibung

Die vorliegende Offenbarung betrifft eine Befestigungseinheit zur fadenlosen Befestigung von Gewebe an Knochen, mit einem Ankerelement, das an einem Knochen fixierbar ist, und einem Halteelement, das mit dem Ankerelement koppelbar ist, um einen Gewebeabschnitt am Knochen anzulagern, wobei das Ankerelement und das Halteelement miteinander verrastbar sind und zwischen sich einen Haltebereich ausbilden, in dem der Gewebeabschnitt aufnehmbar ist.

Aus der US 5,013,316 A ist ein Verankerungssystem zur Befestigung von Weichgewebe bekannt, mit einem Fußbolzen zur Befestigung in einem Knochen, wobei der Fußbolzen eine mittige Ausnehmung zur Aufnahme eines Halteelements aufweist, wobei das Halteelement mit dem Fußbolzen verrastbar ist.

Aus der US 6,056,751 A ist eine Weichgewebe-Fixierungsanordnung für die nahtlose Befestigung von Weichgewebe an Knochen bekannt, wobei die Anordnung ein Knochenverankerungselement und ein Verbindungselement aufweist, wobei das Knochenverankerungselement für eine Befestigung in einem Loch in einem Knochen ausgebildet ist und einen Verankerungsabschnitt an einem vorderen Ende und einen Treibabschnitt an einem Treibende aufweist, und wobei das Knochenverankerungselement und das Verbindungselement für einen Passeingriff an einer Schnittstelle ausgebildet sind.

Aus der US 6,162,234 A ist ein Befestigungssystem zur Befestigung eines Gewebes an einem anderen Gewebe bekannt, mit einem ersten langgestreckten Befestigungselement, das an einem Ende einer Bohrung in einem ersten Gewebe befestigt werden kann, wobei das langgestreckte Befestigungselement einen sich über die Bohrung hinaus erstreckenden Gewindeverlängerungsbereich aufweist, mit einer geteilten Spannzange, die eine innere Gewindeöffnung aufweist, und mit einer Hülse, die unter Kraftaufbringung über die geteilte Spannzange in ihre Position auf dem Verlängerungsbereich des Verbindungselements gleitet, um die geteilte Spannzange gegen das Befestigungselement zu klemmen sowie um die geteilte Spannzange zu verriegeln.

Aus der DE 299 22 088 U1 ist eine Vorrichtung zur Befestigung eines Kreuzbandes an einem Knochen bekannt, mit einer Hohlschraube zum Einschrauben in den Knochen und mit einem Klemmteller, wobei die Hohlschraube einen Kopf aufweist, der dem Klemmteller zugewandt ist, und wobei der Klemmteller mittels einer Befestigungsschraube gegen den Kopf der Hohlschraube verspannbar ist.

Aus der DE 602 17 981 T2 ist eine Vorrichtung zum Anbringen eines Gewebegerüsts an Knochengewebe bekannt, umfassend eine Plattform, einen Stift, der sich von der Plattform nach distal erstreckt, wobei der Stift in ein im Knochengewebe ausgebildetes Loch einsetzbar ist, und mindestens einen am Stift ausgebildeten Rasthaken, der den Stift im Knochengewebe fixieren kann.

Befestigungssysteme zur Fixierung von Weichgewebe an Knochen, etwa zur Fixierung von Muskeln, Bändern oder dergleichen, sind bekannt. Beispielhaft wird auf Verfahren zur Fixierung der Rotatorenmanschette an Oberarmknochen verwiesen. Allgemein können derartige Befestigungssysteme bei verschiedenen arthroskopischen, endoskopischen und offenen chirurgischen Behandlungen genutzt werden.

Die Befestigungssysteme umfassen üblicherweise ein Ankerelement, das etwa als Schlaganker oder Schraubanker ausgestaltet ist. Ferner umfassen herkömmliche Befestigungssysteme üblicherweise einen Faden, der durch das zu fixierende Gewebe durchgestoßen wird, etwa mit einer Nadel oder dergleichen. Es sind Befestigungssysteme bekannt, bei denen ein solcher Faden dann direkt oder indirekt mit dem Anker verknotet wird. Ferner sind sogenannte knotenlose Befestigungssysteme bekannt, die Fäden zur Befestigung nutzen, die jedoch geklemmt werden, so dass kein Knoten erforderlich ist.

Beispielhaft ist aus der DE 10 2006 010 116 A1 ein Ankerelement zum knotenfreien Fixieren von Gewebe an einem Knochen mittels zumindest eines durch das Ankerelement hindurchgefädelten Fadens bekannt, mit einem Körper, an dessen Außenseite Vorsprünge vorgesehen sind, die ein Abziehen des in den Knochen eingebrachten Ankerelements verhindern, mit einer im distalen Endbereich des Körpers angeordneten und durch diesen hindurchreichenden Querbohrung zum Durchfädeln des zumindest einen Fadens quer durch den Körper, und mit einem längs des Körpers bewegbaren Klemmelements zum Klemmen des durch den Körper hindurchgefädelten Fadens.

Das Ankerelement erlaubt eine fadenbasierte, jedoch knotenlose Befestigung des Gewebes am Knochen. Gleichwohl ist es erforderlich, zumindest drei separate Befestigungselemente miteinander zu verbinden, nämlich den eigentlichen Anker, das Klemmelement sowie den zwischengeschobenen Faden. Es ist anzumerken, dass der Faden ösenartig gelegt sein muss, um das Gewebe greifen und sichern zu können.

Aus der WO 01/80751 A1 ist eine fadenlose Einheit zum Fixieren von Knochen mit einem axial stauchbaren Ankerelement bekannt, das an einem Stift aufgenommen ist, der in einer Hülse geführt ist, die in den Knochen eingebracht ist. Wesentliche Ausführungsformen der Offenbarung der WO 01/80751 A1 beziehen sich auf Einheiten, die durch ein Durchgangsloch im Knochen vollständig durch den Knochen hindurch geführt werden.

Im Bereich der Arthroskopie und der Endoskopie, generell im Bereich der minimalinvasiven Chirurgie, wird angestrebt, mögliche Traumata im Körper des Patienten zu minimieren oder gar gänzlich zu unterbinden. Vorzugsweise werden möglichst kleine Zugangsöffnungen zum Körper geschaffen, durch die hindurch jedoch auch die Handhabung der beteiligten Komponenten erfolgen muss. Dies betrifft etwa auch die vorstehend beschriebenen Befestigungssysteme.

Bei fadenbasierten Befestigungssystemen zum Fixieren von Gewebe an Knochen ist es zuweilen erforderlich, mehrere Fäden und mehrere Anker zu verwenden. Ferner ist es zuweilen erforderlich, einen Faden mehrmals durch einen Gewebeabschnitt zu führen, um eine bessere Krafteinleitung zu ermöglichen. Darüber hinaus müssen die Fäden aufgrund des kleinen Raums außerhalb des Körpers geknotet und dann wieder durch entsprechende Kanülen eingeführt werden.

Der vorliegende Offenbarung liegt die Aufgabe zugrunde, eine Befestigungseinheit zur fadenlosen Befestigung von Gewebe an Knochen anzugeben, die eine noch einfachere Handhabbarkeit ermöglicht. Ferner soll der Vorgang der Fixierung des Gewebes möglichst atraumatisch (traumaarm oder traumafrei) vonstattengehen können. Vorzugsweise sind mit der Befestigungseinheit hohe Haltekräfte erzielbar, wobei die Flächenbelastung am Gewebe nicht übermäßig groß sein sollte. Das Schaffen und die Sicherung der Verbindung soll möglichst einfach und fehlerarm erfolgen. Die Befestigungseinheit soll möglichst nur eine geringe Anfälligkeit für Bedienerfehler aufweisen.

Ferner soll das Gewebe mit möglichst geringem Raumbedarf für die Handhabung der beteiligten Elemente am Knochen fixiert werden können. Schließlich soll nach Möglichkeit die Zahl der Zugangsstellen zum Körper bzw. die Größe der erforderlichen Öffnungen reduziert werden.

Ferner soll eine Handhabungsvorrichtung angegeben werden, die mit einer solchen Befestigungseinheit versehen ist und das Fixieren des Gewebeabschnitts mit geringem Aufwand ermöglicht. Schließlich soll ein Revisionswerkzeug angegeben werden, mit dem die Verbindung möglichst traumaarm oder traumafrei wieder gelöst werden kann.

Diese Aufgabe wird bei einer Befestigungseinheit der eingangs genannten Art dadurch gelöst, dass am Ankerelement ein Verbindungsdorn ausgebildet ist, der sich zwischen einem Grundkörper des Halteelements und einem Schaft des Ankerelements erstreckt, dass der Verbindungsdorn dazu ausgebildet ist, den Gewebeabschnitt zu durchragen, wenn das Halteelement und das Ankerelement aufeinander zu bewegt werden, dass der Verbindungsdorn mit einer Riffelung oder Umfangskerbung versehen ist, die eine Lagesicherung im verrasteten Zustand bewirkt, und dass am Halteelement eine Rastausnehmung ausgebildet ist, die im verrasteten Zustand vom Verbindungsdorn zumindest abschnittsweise durchragt wird.

Die Aufgabe der Erfindung wird auf diese Weise vollkommen gelöst.

Erfindungsgemäß wird nämlich in einfacher Weise eine Rastverbindung geschaffen, die einfach durch Druck auf das Halteelement in Richtung auf das Ankerelement erzeugbar ist. Mit anderen Worten erfolgt die Verrastung des Ankerelements mit dem Halteelement durch eine Bewegung, die richtungsgleich zur Zuführbewegung des Ankerelements und des Halteelements ist. Dies vereinfacht die Montage. Ferner ist kein übermäßig großer Raum zum Setzen einer solchen Verbindung erforderlich.

Eine definierte Druckbewegung auf das Halteelement verbindet dieses vorzugsweise unlösbar mit dem Ankerelement. Ähnlich einem Druckknopf oder einer ähnlichen formschlüssigen und/oder kraftschlüssigen Pressverbindung lassen sich grundsätzlich unlösbare Verbindungen schaffen. Da das Ankerelement und das Halteelement flächig miteinander zusammenwirken und zwischen sich den Gewebeabschnitt einklemmen können, sind einerseits hohe Kräfte übertragbar. Dies geht jedoch nur mit einer geringen traumatischen Belastung des Gewebeabschnitts einher. Insbesondere die Flächenbelastung am Gewebeabschnitt sinkt, verglichen mit der Fadenfixierung, deutlich.

Ein weiterer Vorteil der Rastverbindung besteht darin, dass mehrere Raststufen vorgesehen sein können, so dass die Befestigungseinheit flexibel an Gewebeabschnitte verschiedener Dicke/Stärke adaptierbar ist.

Das Ankerelement und/oder das Halteelement können grundsätzlich aus resorbierbaren (abbaubaren) oder nicht resorbierbaren Materialien bestehen. Denkbar sind etwa Metalle, Nicht-Metalle, Kunststoffe, Keramik, etc. Auch mit Werkstoffen, die keine hohe spezifische Festigkeit aufweisen, lassen sich sichere Verbindung schaffen, da Kräfte flächenmäßig aufgenommen werden. Die spezifische Belastung sinkt deutlich.

Gemäß einer beispielhaften Ausgestaltung ist das Halteelement knopfartig oder tellerartig gestaltet und mit einer dem Ankerelement zugewandte Kontaktfläche versehen, die dazu ausgebildet ist, im verrasteten Zustand den Gewebeabschnitt in Richtung auf das Ankerelement zu beaufschlagen, wobei die Kontaktfläche vorzugsweise zumindest abschnittsweise mit Erhebungen versehen ist.

Diese Maßnahme hat den Vorteil, dass die effektiv wirksame Kontaktfläche weiter vergrößert werden kann. Ferner lässt sich die Belastung des Gewebeabschnitts zwischen dem Ankerelement und dem Halteelement flächig verteilen. Die Erhebungen können etwa als Wellen, Dome, Wulste oder dergleichen gestaltet sein. Die Erhebungen können ein Muster umfassen.

Gemäß einer beispielhaften Ausgestaltung weist das Ankerelement eine Gegenfläche auf, die der Kontaktfläche zugewandt ist, wobei die Gegenfläche und die Kontaktfläche im verrasteten Zustand zwischen sich den Haltebereich für den Gewebeabschnitt definieren.

Demgemäß kann zwischen der Gegenfläche des Ankerelements und der Kontaktfläche des Halteelements ein Formschlussbereich oder ein Labyrinth gebildet sein. Selbst dann, wenn die Gegenfläche und die Kontaktfläche einander nicht unmittelbar kontaktieren. Da der Gewebeabschnitt dazwischen aufgenommen ist, lassen sich die Haltekräfte deutlich erhöhen. Der Gewebeabschnitt ist zumindest teilweise auch formschlüssig lagegesichert. Ein weiterer Vorteil dieser Gestaltung ist, dass lokale Extrembelastungen am Gewebe reduziert oder sogar gänzlich vermieden werden.

Beispielhaft können die Gegenfläche und die Kontaktfläche aneinander angepasste Muster aufweisen, wobei Erhebungen auf der einen Seite mit Senkungen auf der anderen Seite korrespondieren.

Gemäß einer beispielhaften Ausgestaltung ist die Gegenfläche zumindest abschnittsweise mit Erhebungen versehen, die an Erhebungen der Kontaktfläche des Halteelements angepasst sind, so dass sich im verrasteten Zustand ein Labyrinth oder ein Muster aus Erhebungen der Kontaktfläche und Erhebungen der Gegenfläche ergibt. Vorzugsweise sind also die Kontaktfläche und die Gegenfläche zumindest abschnittsweise uneben gestaltet. Dies kann jedoch eine organische Gestaltung umfassen, die weiche oder tangentiale Übergänge zwischen einzelnen Gestaltelementen (Erhebungen, Senkungen und dergleichen) umfasst.

Beispielhaft kann sich eine wellenartige Gestaltung, umfassend Berge und Täler, auf der Kontaktfläche und der Gegenfläche ergeben. Berge und Täler der Welle können sich grundsätzlich in radialer Richtung oder in Kreisrichtung (Umfangsrichtung) erstrecken.

Ein Vorteil der Verrastung des Ankerelements und des Halteelements besteht darin, dass die Kontaktfläche und die Gegenfläche während des Fügevorgangs aufeinander zubewegt werden, aber nicht in anderer Weise relativ zueinander bewegt werden, etwa gegeneinander verdreht werden. Dies hat den Vorteil, dass die gewünschte Relativausrichtung, betreffend die Oberflächengestaltung der Kontaktfläche und der Gegenfläche, beibehalten wird.

In diesem Zusammenhang kann es von Vorteil sein, das Ankerelement und das Halteelement derartig zu gestalten, dass diese verdrehgesichert relativ zueinander angeordnet sind. Mit anderen Worten können das Ankerelement und das Halteelement zwar gemeinsam absolut verdreht werden. Jedoch ist gemäß zumindest dieser Ausgestaltung keine Relativverdrehung zwischen dem Ankerelement und dem Halteelement möglich.

Gemäß einer beispielhaften Ausgestaltung ist eine Rastverbindung vorgesehen, die eine Annäherung zwischen dem Halteelement und dem Ankerelement erlaubt und im verrasteten Zustand einer Lösebewegung zwischen dem Halteelement und dem Ankerelement entgegenwirkt.

Die Rastverbindung kann etwa ein Gestaltelement umfassen, das tannenbaumartig gestaltet ist. Mit anderen Worten ist die Rastverbindung etwa ähnlich einem Nagelanker gestaltet. Allgemein kann die Rastverbindung Rastelemente, etwa Rastzähne, Rastfedern, Widerhaken und Ähnliches umfassen. Demgemäß kann die Rastverbindung eine Zuführbewegung, also eine erlaubte Bewegung, und eine entgegengesetzte Sperrrichtung definieren, in der keine Lösebewegung ermöglicht ist.

Ferner ist ein Verbindungsdorn vorgesehen, der sich zwischen einem Grundkörper des Halteelements und einem Schaft des Ankerelements erstreckt, wobei der Verbindungsdorn vorzugsweise dazu ausgebildet ist, den Gewebeabschnitt zu durchragen, wenn das Halteelement und das Ankerelement aufeinander zu bewegt werden.

Der Verbindungsdorn kann etwa tannenbaumartig gestaltet sein und sich grundsätzlich in Richtung auf seine Spitze verjüngen. Der Verbindungsdorn ist dazu ausgebildet, das zu fixierende Gewebe zu durchstechen. Auf diese Weise dient der Verbindungsdorn einerseits dazu, den Gewebeabschnitt zumindest teilweise festzulegen. Zum anderen verbindet der Verbindungsdorn das Halteelement und das Ankerelement.

Ferner ist der Verbindungsdorn mit einer Riffelung oder Umfangskerbung versehen, die eine Lagesicherung im verrasteten Zustand bewirkt. Demgemäß kann der Verbindungsdorn einen Bestandteil der Rastverbindung ausbilden. Der Verbindungsdorn kann etwa tannenartig gestaltet sein und über eine Umfangsrändelung verfügen. Allgemein kann der Verbindungsdorn ähnlich einem Ankernagel gestaltet sein. Es ist auch vorstellbar, anders gestaltete Zacken, Zähne oder dergleichen am Verbindungsdorn vorzusehen.

Gemäß einer beispielhaften Ausgestaltung wirkt der Verbindungsdorn mit einer Rastausnehmung zusammen, die Rastelemente aufweist, die vorzugsweise richtungsabhängig gestaltet sind und eine Sperrrichtung aufweisen.

Vorzugsweise sind die Rastelemente als Rastfedern oder Rastnasen ausgestaltet. Die Rastelemente können mit der Umfangskerbung des Verbindungsdorns verrastet werden, wenn das Halteelement und das Ankerelement aufeinander zu bewegt werden. In einer entgegengesetzten Sperrrichtung verhindern die Rastelemente formschlüssig und/oder kraftschlüssig das Lösen der Verbindung. Demgemäß ist die Montage, also die Verrastung relativ leicht möglich. Die Demontage ist umso schwieriger. Es kann vorgesehen sein, dass eine Demontage nur durch Zerstörung der Rastelemente ermöglicht ist. Die Rastelemente können grundsätzlich auch als Schnappelemente bezeichnet werden. Demgemäß kann die sich ergebende Rastverbindung auch als Schnappverbindung bezeichnet werden.

Ferner ist der Verbindungsdorn am Ankerelement ausgebildet, insbesondere als Fortsatz in Richtung auf das Halteelement, wobei am Halteelement eine Rastausnehmung ausgebildet ist, die im verrasteten Zustand vom Verbindungsdorn zumindest abschnittsweise durchragt wird. Gemäß dieser Ausgestaltung erstreckt sich der Verbindungsdorn von distal nach proximal. Das Halteelement kann auf den Verbindungsdorn aufgesetzt werden.

Gemäß einer alternativen Ausgestaltung ist der Verbindungsdorn am Halteelement ausgebildet, insbesondere als Fortsatz in Richtung auf das Ankerelement, wobei am Ankerelement eine Rastausnehmung ausgebildet ist, die im verrasteten Zustand vom Verbindungsdorn zumindest abschnittsweise durchragt wird. Gemäß dieser Ausgestaltung erstreckt sich der Verbindungsdorn von proximal nach distal. Der am Halteelement ausgebildete Verbindungsdorn kann in die am Ankerelement vorgesehene Rastausnehmung eingeführt werden. Dies umfasst das Durchführen oder Durchstechen des Gewebeabschnitts.

Gemäß einer beispielhaften Ausgestaltung ist am Ankerelement ein Führungsstift angeordnet, der als Montagehilfe ausgestaltet ist und sich in Richtung auf das Halteelement erstreckt, wobei der Verbindungsdorn eine Führungsausnehmung aufweist, in die der Führungsstift einführbar ist, um den Verbindungsdorn der Rastausnehmung im Ankerelement zuzuführen.

Dies hat den Vorteil, dass der Verbindungsdorn, der gemäß dieser Variante am Halteelement ausgebildet ist, nicht "blind" in die Rastausnehmung eingeführt werden muss. Wenn das Ankerelement gesetzt ist, wird der Gewebeabschnitt zumindest teilweise darüber gelegt. Dies bedeutet, dass der Gewebeabschnitt das Ankerelement teilweise oder vollständig überdecken kann. Da jedoch der Führungsstift durch den Gewebeabschnitt durchgestochen werden kann, ist eine Orientierungshilfe oder Positionierhilfe vorgesehen. Der Verbindungsdorn, der wiederum mit einer Führungsausnehmung versehen ist, kann auf den Führungsstift aufgeschoben werden und schlussendlich in die Rastausnehmung eingeführt werden.

Vorzugsweise ist der Führungsstift dazu ausgebildet, nach dem Rastvorgang gelöst und entfernt zu werden. Der Führungsstift kann als separates Teil gestaltet sein, das lösbar am Ankerelement aufgenommen ist. Es ist jedoch auch vorstellbar, das Ankerelement und den Verbindungsdorn als integral gestaltetes Bauteil auszuführen. Demgemäß ist beispielhaft eine Sollbruchstelle vorgesehen, so dass der Führungsstift definiert lösbar und entfernbar ist.

Ein weiterer Aspekt der vorliegenden Offenbarung betritt eine Handhabungsvorrichtung zum Einbringen einer Befestigungseinheit, die Folgendes aufweist:
- ein Montagewerkzeug, und
- eine Befestigungseinheit gemäß zumindest einer hierin genannten Ausführungsform,
   wobei das Montagewerkzeug eine Schaftbaugruppe mit einem distalen Aufnahmeabschnitt aufweist, an dem die Befestigungseinheit aufnehmbar ist,
   wobei die Schaftbaugruppe eine erste Schaftkomponente zur Aufnahme des Ankerelements und eine zweite Schaftkomponente zur Aufnahme des Halteelements aufweist,
   wobei das Ankerelement und das Halteelement in einem voneinander entkoppelten Zustand axial versetzt zueinander aufnehmbar sind,
   wobei die erste Schaftkomponente dazu ausgebildet ist, das Ankerelement dem Knochen zuzuführen, und
   wobei die zweite Schaftkomponente dazu ausgebildet ist, das Halteelement und das Ankerelement miteinander zu verrasten, wenn das Ankerelement fixiert ist.

Das Montagewerkzeug der Handhabungsvorrichtung erlaubt eine gemeinsame Zuführung des Ankerelements und des Halteelements, wobei diese jedoch noch fest nicht miteinander verbunden sind. Das Montagewerkzeug ist dazu ausgestaltet, zunächst das Ankerelement einzutreiben, etwa durch entsprechenden Druck (Schlaganker) oder durch eine Schraubbewegung (Schraubanker). Da das Ankerelement und das Halteelement durch verschiedene Schaftkomponenten gehalten werden, ist es grundsätzlich möglich, das Ankerelement und das Halteelement relativ zueinander zu bewegen. Insbesondere kann das Halteelement auf das Ankerelement zu bewegt werden, um die Rastverbindung zu schaffen.

Die Zuführrichtung für das Ankerelement und das Halteelement ist identisch. Insbesondere muss die Schaftbaugruppe des Montagewerkzeugs, zumindest gemäß einigen Ausführungsformen, lediglich einmal in den Körper eingeführt werden. Es versteht sich, dass das Montagewerkzeug nach dem Setzen des Ankerelements und dem Fixieren des Ankerelements am Knochen zunächst zurückgesetzt, also vom Ankerelement abgehoben wird. Dies ist erforderlich, um den zu fixierenden Gewebeabschnitt zwischen dem Ankerelement und dem Halteelement zu platzieren. Anschließend wird das Montagewerkzeug wiederum in Richtung auf die Verbindungsstelle (also von proximal nach distal) geführt, um das Halteelement dem Ankerelement zuzuführen. Dies kann ein Durchstechen des Gewebeabschnitts umfassen.

Gemäß einer beispielhaften Ausgestaltung der Handhabungsvorrichtung ist die erste Schaftkomponente dazu ausbildet, zumindest eine Vorschubkraft oder ein Drehmoment auf das Ankerelement zu übertragen, wobei die zweite Schaftkomponente relativ zur ersten Schaftkomponente verschiebbar ist, um das Halteelement in Richtung auf das Ankerelement zu bewegen.

Gemäß dieser Ausgestaltung ist die erste Schaftkomponente beispielhaft als Hohlschaft ausgestaltet. Die zweite Schaftkomponente ist etwa innerhalb der ersten Schaftkomponente angeordnet. Beide Schaftkomponenten sind gemäß zumindest einigen Ausführungsformen relativ zueinander axial verschiebbar. Demgemäß kann zunächst die erste Schaftkomponente auf das Ankerelement einwirken, um dieses zu fixieren. Anschließend kann das Halteelement montiert werden.

Gemäß einer beispielhaften Ausgestaltung weist die Handhabungsvorrichtung ferner eine Zugeinheit auf, die dazu ausgebildet ist, einen Führungsstift, der am Ankerelement angeordnet ist, vom Ankerelement zu lösen, wenn das Halteelement mit dem Ankerelement gekoppelt ist, wobei die Zugeinheit vorzugsweise der Schaftbaugruppe zugeordnet ist. Die Schaftbaugruppe kann als Träger für die Zugeinheit gestaltet sein. Die Zugeinheit kann in der Schaftbaugruppe aufnehmbar sein.

Auf diese Weise kann mit lediglich einem einzigen Montagewerkzeug das Ankerelement fixiert werden, das Halteelement mit dem Ankerelement rastend verbunden werden und der Führungsstift gelöst werden. Diese Ausführungsform des Montagewerkzeugs bietet sich insbesondere bei der Ausgestaltung der Befestigungseinheit an, bei der der Verbindungsdorn am Halteelement angeordnet ist und sich in Richtung auf das Ankerelement erstreckt, wobei am Ankerelement der Führungsstift als Montagehilfe ausgebildet ist. Es versteht sich, dass diverse beispielhafte Ausgestaltungen denkbar sind, die keinen Führungsstift erfordern.

Ein weiterer Aspekt der Offenbarung betrifft ein Revisionswerkzeug für eine Handhabungsvorrichtung, wobei das Revisionswerkzeug zum Lösen des Halteelements vom Ankerelement ausgebildet ist, und wobei das Revisionswerkzeug als Abziehwerkzeug oder als Sprengwerkzeug gestaltet ist.

Somit kann das Revisionswerkzeug etwa ähnlich einem Abzieher oder einem Mutternsprenger gestaltet sein. Vorzugsweise ist das Revisionswerkzeug dazu ausgestaltet, das Halteelement radial zu klemmen und/oder radial zu hintergreifen/untergreifen, um das Halteelement möglichst ohne traumatische Auswirkungen auf das Gewebe lösen zu können. Beispielhaft sind am Halteelement seitliche Ausnehmungen vorgesehen, in die das Revisionswerkzeug, insbesondere ein Greiferelement oder Klemmelement, eingreifen kann.

Jedoch ist es auch vorstellbar, vorrangig kraftschlüssig am Halteelement anzugreifen. Ferner ist es vorstellbar, das Halteelement zumindest teilweise zu verformen, um die Lösekräfte aufbringen zu können. Gemäß zumindest einigen Ausführungsformen umfasst das Revisionswerkzeug einen Niederhalter, der mittelbar oder unmittelbar auf den am Ankerelement angeordneten Verbindungsdorn einwirkt und als Abstützung dient. Auf diese Weise können hohe Kräfte auf das Halteelement aufgebracht werden, um das Halteelement zu lösen. Dies wirkt sich jedoch nicht nachteilig auf die Position des Ankerelements aus, das gewissermaßen durch den Niederhalter entkoppelt ist. Auf diese Weise können die Haltekräfte oder Rastkräfte der unidirektional wirksamen Rastverbindung überwunden werden.

Es versteht sich, dass die vorstehend genannten und die nachstehend noch zu erläuternden Merkmale der Offenbarung nicht nur in der jeweils angegebenen Kombination, sondern auch in anderen Kombinationen oder in Alleinstellung verwendbar sind, ohne den Rahmen der vorliegenden Offenbarung zu verlassen.

Weitere Merkmale und Vorteile der Offenbarung ergeben sich aus der nachfolgenden Beschreibung mehrerer bevorzugter Ausführungsbespiele unter Bezugnahme auf die Zeichnungen. *Die* *Figuren 6 bis 9* *zeigen keine Verkörperungen der im unabhängigen Anspruch beanspruchten Erfindung. Es zeigen:*
- Fig. 1: eine schematische Ansicht zur Veranschaulichung eines Anwendungsbereichs für eine Befestigungseinheit zur fadenlosen Befestigung von Gewebe an einem Knochen;
- Fig. 2: eine perspektivische Seitenansicht eines Halteelements einer Befestigungseinheit;
- Fig. 3: eine perspektivische Ansicht eines Ankerelements einer Befestigungseinheit, wobei in den Fig. 2 und 3 unterschiedliche Maßstäbe gewählt wurden;
- Fig. 4: einen seitlichen Schnitt durch eine montierte Befestigungseinheit, bei der ein Halteelement gemäß Fig. 2 mit einem Ankerelementgemäß Fig. 3 verrastet ist;
- Fig. 5: eine vergrößerte Teildarstellung der Anordnung gemäß Fig. 4 im Bereich einer Rastverbindung zwischen dem Ankerelement und dem Halteelement;
- Fig. 6: eine perspektivische Seitenansicht eines Halteelements einer Befestigungseinheit;
- Fig. 7: eine perspektivische Ansicht eines Ankerelements einer Befestigungseinheit, wobei in den Fig. 6 und 7 unterschiedliche Maßstäbe gewählt wurden;
- Fig. 8: einen seitlichen Schnitt durch eine montierte Befestigungseinheit, bei der ein Halteelement gemäß Fig. 6 mit einem Ankerelement gemäß Fig. 7 verrastet ist;
- Fig. 9: eine vergrößerte Teildarstellung der Anordnung gemäß Fig. 8 im Bereich einer Rastverbindung zwischen dem Ankerelement und dem Halteelement;
- Fig. 10: eine schematische Seitenansicht einer weiteren Befestigungseinheit mit einem Ankerelement und einem Halteelement, die im nicht verrasteten Zustand gezeigt sind, zur Veranschaulichung von Kontaktflächen;
- Fig. 11: eine perspektivische frontale Ansicht einer Handhabungsvorrichtung mit einem Montagewerkzeug zum Einbringen einer Befestigungseinheit;
- Fig. 12: eine geschnittene Teilansicht eines Aufnahmeabschnitts eines Montagewerkzeugs, an dem eine Befestigungseinheit aufgenommen ist, in einem ersten Zustand;
- Fig. 13: eine weitere Ansicht der Anordnung gemäß Fig. 12 in einem zweiten Zustand;
- Fig. 14: eine geschnittene Teilansicht eines Aufnahmeabschnitts einer weiteren Ausführungsform eines Montagewerkzeugs, an dem eine Befestigungseinheit aufgenommen ist; und
- Fig. 15: eine geschnittene Teilansicht eines Revisionswerkzeugs zum Lösen eines Halteelements von einem Ankerelement.

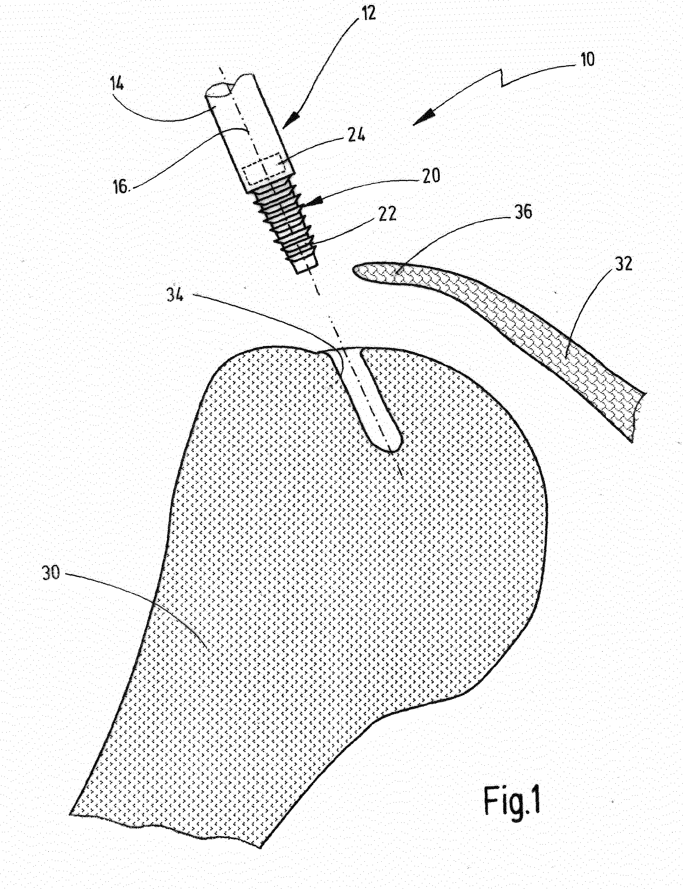

Fig. 1 veranschaulicht anhand einer schematischen, stark vereinfachten Teilansicht eine Handhabungsvorrichtung 10, die zur Befestigung von Weichgewebe an Knochen nutzbar ist.

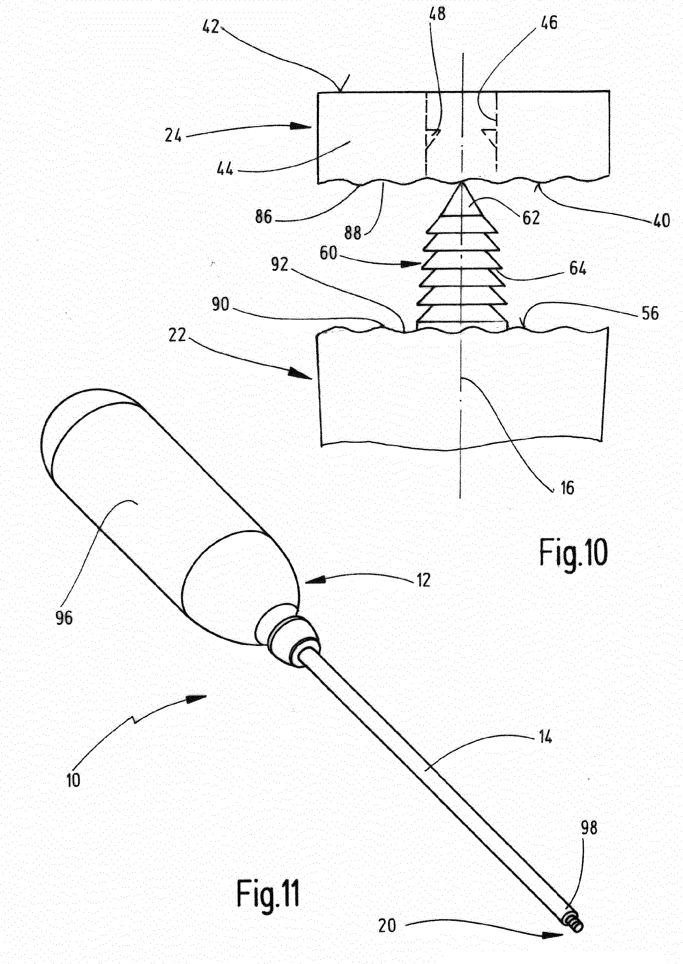

Die Vorrichtung 10 umfasst ein Montagewerkzeug 12, wobei in Fig. 1 lediglich ein distaler Abschnitt einer Schaftbaugruppe 14 des Montagewerkzeugs 12 dargestellt ist. An einem proximalen Ende des Montagewerkzeugs 12 befindet sich üblicherweise ein Handstück, vergleiche etwa auch Fig. 11.

An der Schaftbaugruppe 14 ist eine Befestigungseinheit 20 aufgenommen, die in Fig. 1 lediglich symbolhaft dargestellt ist. Die Schaftbaugruppe 14 und die Befestigungseinheit 20 sind entlang einer gemeinsamen Längsachse 16 ausgerichtet. Die Befestigungseinheit 20 umfasst ein Ankerelement 22 sowie ein Halteelement 24. Das Ankerelement 22 ragt am distalen Ende der Schaftbaugruppe 14 über diese hinaus. Das Halteelement 24 ist proximal vom Ankerelement 22 angeordnet. Das Ankerelement 22 kann grundsätzlich als Schlaganker oder Schraubanker ausgestaltet sein.

Fig. 1 zeigt ferner anhand einer schematischen Teilschnittansicht einen Knochen 30 sowie vom Knochen 30 gelöstes Gewebe 32. Bei dem Gewebe 32 kann es sich beispielhaft um einen Abschnitt der sogenannten Rotatorenmanschette handeln. Demgemäß handelt es sich bei dem Knochen 30 um einen Oberarmknochen. Im gesunden Zustand ist das Gewebe 32 auf natürliche Weise am Knochen 30 fixiert. Am Gewebe 32 können jedoch Beschädigungen auftreten, etwa Risse oder dergleichen. Dies kann dazu führen, dass ein Gewebeabschnitt 36 vom Knochen 30 gelöst wird.

Der Gewebeabschnitt 36 kann am Knochen 30 fixiert werden, wenn das Ankerelement 22 in eine Halteausnehmung 34 am Knochen 30 eingebracht wird. Dies heißt mit anderen Worten, dass der Knochen 30 zumindest abschnittsweise freigelegt wird, um die Halteausnehmung 34 etwa mittels Bohren und/oder Fräsen zu erzeugen. Der zu fixierende Gewebeabschnitt 36 wird abschnittsweise umgeklappt oder beiseite geschoben. Hiernach kann das Ankerelement 22 in der Halteausnehmung 34 fixiert werden. Danach kann der Gewebeabschnitt 36 unter Hinzunahme des Halteelements 24 am Knochen 30 festgelegt werden, wenn das Halteelement 24 in geeigneter Weise mit dem Ankerelement 22 verbunden wird.

Gemäß einer beispielhaften Ausführungsform handelt es sich bei der Halteausnehmung 34 um ein Sackloch. D.h., die Halteausnehmung 34 ist gemäß dieser Ausführungsform nicht als Durchgangsloch gestaltet, das sich vollständig durch den Knochen 30 hindurch erstreckt. Diese Gestaltung bedingt, dass das Ankerelement 22 ungeachtet der Ausführung als Schlaganker oder Schraubanker für das Einführen in Sacklöcher dazu ausgestaltet ist, eine sichere Befestigung des Gewebeabschnitts 36 zu gewährleisten.

Mit anderen Worten ist im fixierten Zustand der Gewebeabschnitt 36 zwischen dem Ankerelement 22 und dem Halteelement 24 aufgenommen.

Um das Trauma beim Fixieren des Gewebeabschnitts 36 am Knochen 30 möglichst klein zu halten, werden minimalinvasive arthroskopische und/oder endoskopische Verfahren genutzt. Es ist wünschenswert, wenn mit lediglich einem Montagewerkzeug 12 sowohl das Ankerelement 22 als auch das Halteelement 24 der Befestigungseinheit 20 der Halteausnehmung 34 zuführbar sind. Somit muss nicht nach dem Setzen des Ankerelements 22 ein zusätzliches Werkzeug eingeführt werden, um das Halteelement 24 dem Ankerelement 22 zuzuführen.

Im Sinne dieser Offenbarung ist unter dem Begriff proximale Seite bzw. proximale Fläche diejenige Seite oder Fläche zu verstehen, die dem Operateur bei der Handhabung der Befestigungseinheit 20 zugewandt ist. Demgemäß ist unter dem Begriff distale Fläche oder distale Seite diejenige Fläche oder Seite zu verstehen, die vom Operateur abgewandt ist. Allgemein sind distale Elemente oder Abschnitte weiter vom Operateur beabstandet als proximale Elemente oder Abschnitte.

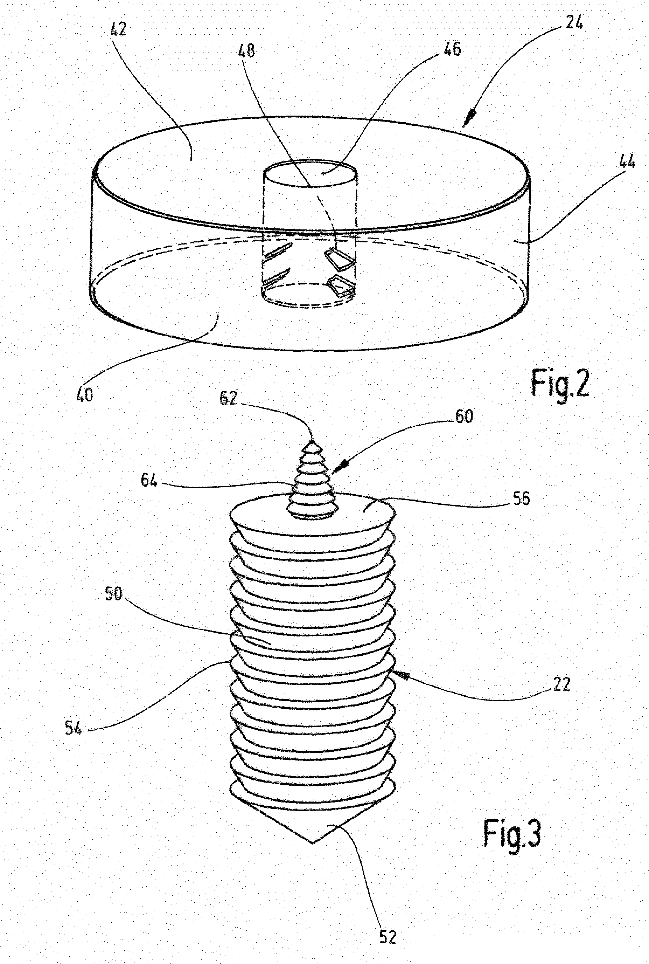

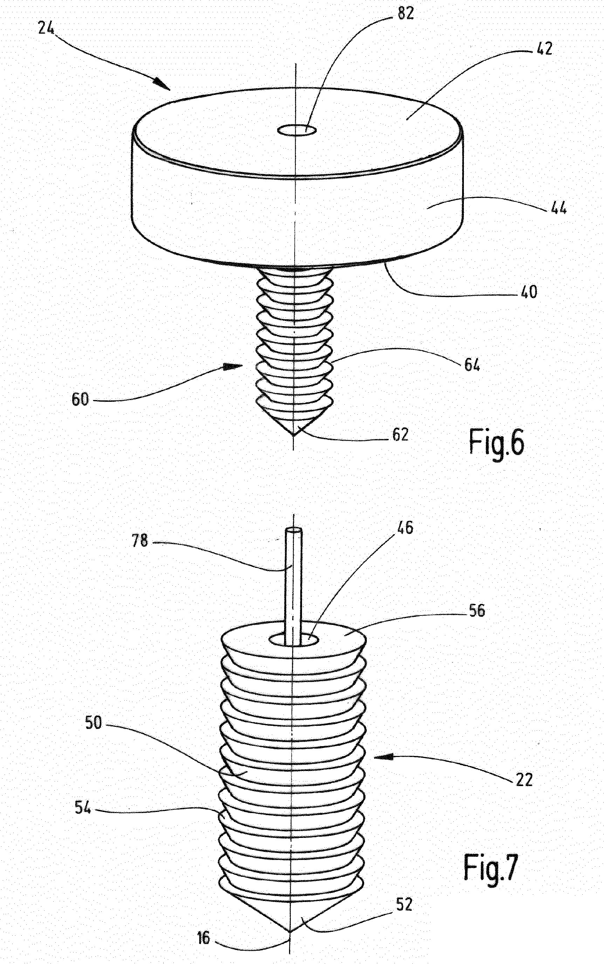

Die in Fig. 1 lediglich schematisch dargestellte Befestigungseinheit 20 mit dem Ankerelement 22 und dem Halteelement 24 wird mit Bezugnahme auf die Fig. 2 bis 5, die eine erste beispielhafte Ausführungsform zeigen, und auf die Fig. 6 bis 9, die eine weitere beispielhafte Ausführungsform zeigen, näher veranschaulicht.

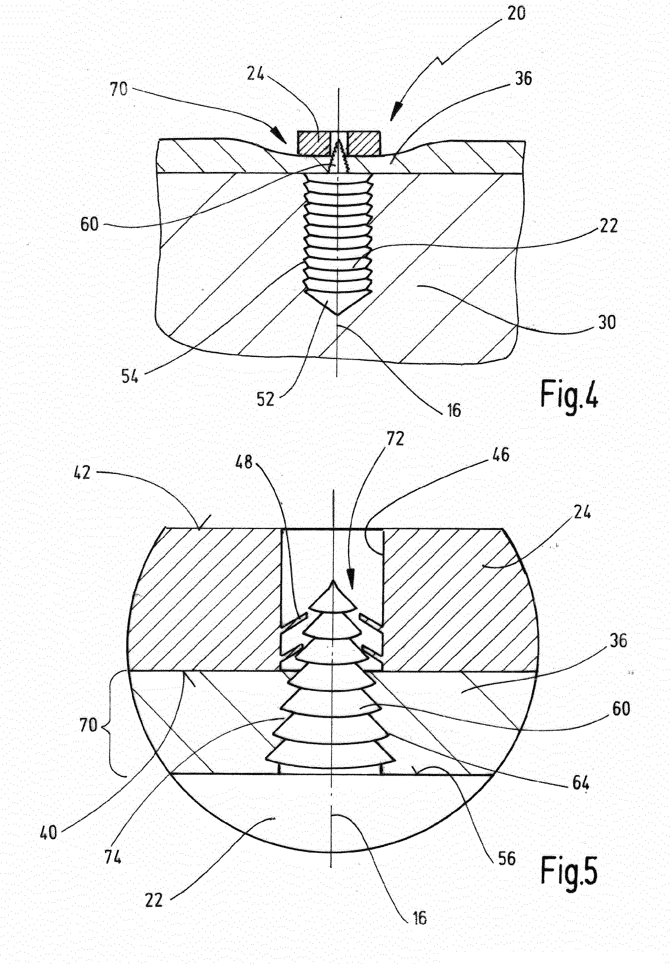

Fig. 1 veranschaulicht eine perspektivische Ansicht eines Halteelements 24 einer Befestigungseinheit 20. Fig. 3 zeigt eine korrespondierende perspektivische Ansicht eines zugeordneten Ankerelements 22. Den Darstellungen in Fig. 2 und 3 liegen keine identischen Maßstäbe zugrunde. Das in Fig. 2 gezeigte Halteelement 24 ist größer als das in Fig. 3 gezeigte Ankerelement 22 dargestellt. Die Fig. 4 und 5 veranschaulichen die Befestigungseinheit 20 mit dem Ankerelement 22 und dem Halteelement 24 im gefügten Zustand, wobei Fig. 5 eine vergrößerte Teilansicht der Darstellung gemäß Fig. 4 ist.

Die Befestigungseinheit 20 ist als fadenlose Befestigungseinheit ausgestaltet. Dies beinhaltet, dass kein separater Faden sowie vorzugsweise kein sonstiges separates Montagehilfsmittel erforderlich ist, um den Gewebeabschnitt 36 am Knochen 30 zu fixieren, vergleiche auch Fig. 1.

Das Halteelement 24 ist knopfartig oder scheibenartig gestaltet. Das Halteelement 24 umfasst eine Kontaktfläche 40, die im gefügten Zustand dem Ankerelement 22 zugewandt ist. Eine von der Kontaktfläche 40 abgewandte Fläche des Halteelements 24 wird als Endfläche 42 bezeichnet. Zwischen der Kontaktfläche 40 und der Endfläche 42 erstreckt sich ein Grundkörper 44 des Halteelements 24. Die Kontaktfläche 40 kann auch als distale Fläche bezeichnet werden. Die Endfläche 42 kann auch als proximale Fläche bezeichnet werden.

Gemäß der anhand der Fig. 2 bis 5 veranschaulichten Ausführungsform umfasst das Halteelement 24 ferner eine Rastausnehmung 46, die den Grundkörper 44 durchragt. Die Rastausnehmung 46 kann auch als mittige Öffnung oder mittige Bohrung bezeichnet werden. In der Rastausnehmung 46 sind Rastelemente 48 angeordnet, die etwa als Rastnasen, Widerhaken oder in ähnlicher Weise ausgestaltet sind. Die Rastelemente 48 sind vorzugsweise richtungsabhängig gestaltet und definieren eine Zuführrichtung, bei der das Halteelement 24 auf das Ankerelement 22 zugeführt wird, und eine Halterichtung oder Rastrichtung, die einem Lösen oder Entfernen des Halteelements 24 vom Ankerelement 22 entspricht.

Gemäß dem in Fig. 2 gezeigten Ausführungsbeispiel sind vier Rastelemente 48 in der Rastausnehmung 46 angeordnet (in Fig. 2 lediglich verdeckt dargestellt). Es versteht sich, dass andere Ausgestaltungen betreffend die Anzahl und/oder Anordnung der Rastelemente 48 denkbar sind.

Fig. 3 veranschaulicht das Ankerelement 22, mit dem das Halteelement 24 zusammenwirkt. Das Ankerelement 22 umfasst einen Schaftkörper 50, an dessen distalem Ende eine Spitze 52 ausgebildet ist. Ferner ist am Schaftkörper 50 eine Befestigungskontur 54 mit Befestigungsvorsprüngen ausgebildet. Demgemäß ist das Ankerelement 22 als Schlaganker ausgestaltet. Es versteht sich, dass die Befestigungskontur 54 gemäß alternativer Ausführungsformen grundsätzlich auch ein Gewinde oder ähnliche Befestigungselemente umfassen kann. Eine Längserstreckung des Ankerelements 22 ist üblicherweise an eine Tiefenerstreckung der in den Knochen 30 eingebrachten Halteausnehmung 34 angepasst.

Am proximalen Ende des Schaftkörpers 50 ist eine Gegenfläche 56 ausgebildet. Im gesetzten Zustand des Ankerelements 22, vergleiche auch Fig. 4 und Fig. 5, schließt die Gegenfläche 56 vorzugsweise bündig oder nahezu bündig mit einer Oberfläche des Knochens 30 ab. Die Gegenfläche 56 ist zumindest im gefügten Zustand der Kontaktfläche 40 des Halteelements 24 zugewandt.

Gemäß der anhand der Fig. 2 bis 5 veranschaulichten Ausführungsform weist das Ankerelement 24 an seinem proximalen Ende ferner einen Verbindungsdorn 60 auf. Der Verbindungsdorn 60 verjüngt sich in Richtung auf die Rastausnehmung 46. Der Verbindungsdorn 60 umfasst eine Spitze 62, die gemäß zumindest einiger Ausführungsbeispiele dazu ausgebildet ist, den Gewebeabschnitt 36 (Fig. 1) zu durchstoßen oder zu durchschneiden, um den Verbindungsdorn 60 durch den Gewebeabschnitt 36 hindurchzuführen.

Ferner umfasst der Verbindungsdorn 60 eine Befestigungskontur oder Rastkontur, beispielhaft in Form einer Umfangskerbung 64. Mit anderen Worten sind am Verbindungsdorn 60 beispielhaft Vorsprünge ausgebildet, die derart an die Rastelemente 48 der Rastausnehmung 46 angepasst sind, dass eine Hinterschnappung oder Verrastung erfolgt, wenn der Verbindungsdorn 60 in die Rastausnehmung 46 eindringt. Auf diese Weise können das Ankerelement 22 und das Halteelement 24 in einfacher Weise mittels einer Schnappverbindung miteinander gefügt werden, ähnlich einem Druckknopf. Der Verbindungsdorn 60 durchragt den zu fixierenden Gewebeabschnitt 36. Der Gewebeabschnitt 36 ist im gefügten Zustand zwischen der Kontaktfläche 40 und der Gegenfläche 56 aufgenommen und somit am Knochen 30 fixiert.

Es ist kein Faden und kein sonstiges Hilfsmittel zum Fixieren des Gewebeabschnitts 36 am Knochen 30 erforderlich. Mittels lediglich zweier Elemente, dem Ankerelement 22 und dem Halteelement 24 kann der Gewebeabschnitt 36 sicher am Knochen gehalten werden, wobei dies mit einem geringen Trauma des Gewebes 32 einhergeht. Der gefügte Zustand der Befestigungseinheit 20 wird mit Bezugnahme auf die Fig. 4 und 5 veranschaulicht.

Das Ankerelement 22 und das Halteelement 24 bilden im verrasteten Zustand einen Haltebereich 70 aus, der sich zwischen der Kontaktfläche 40 und der Gegenfläche 56 erstreckt. Im Haltebereich 70 ist der Gewebeabschnitt 36 aufgenommen. Ein Vorteil dieser Gestaltung ist, dass beim Gewebeabschnitt 36 selbst eine nur relativ geringe Flächenpressung auftritt, so dass insgesamt die punktuelle Belastung deutlich reduziert werden kann.

Fig. 5 veranschaulicht anhand einer vergrößerten Teilansicht eine sich ergebende Rastverbindung 72 zwischen dem Ankerelement 22 und dem Halteelement 24. Die Rastverbindung 72 umfasst eine entsprechende Rastkontur am Verbindungsdorn 60 sowie in der Rastausnehmung 46, beispielhaft Rastelemente 48, die mit einer Umfangskerbung 64, insbesondere mit einzelnen Vorsprüngen davon, zusammenwirken.

Die Gestaltung der Rastverbindung 72 definiert eine Fügerichtung, in der eine Relativbewegung zwischen dem Ankerelement 22 und dem Halteelement 24 entlang der Längsachse 16 grundsätzlich möglich ist. Ferner gibt es eine entgegengesetzte Sperrrichtung, die auch als Löserichtung bezeichnet werden kann. Die Rastelemente 48 wirken einer Bewegung in der Löserichtung hemmend bzw. sperrend entgegen. Die Rastelemente 48 wirken also beispielhaft ähnlich wie eine Anordnung aus Widerhaken.

In Fig. 5 veranschaulicht das Bezugszeichen 74 eine Gewebeöffnung bzw. einen Schnitt, der vom Verbindungsdorn 60 durchragt wird. Vorzugsweise kann die Gewebeöffnung 74 durch den Verbindungsdorn 60 selbst, insbesondere durch dessen Spitze 62 (Fig. 3) selbst erzeugt werden. Mit anderen Worten kann der Verbindungsdorn 60 durch den Gewebeabschnitt 36 hindurchgedrückt werden, um die Gewebeöffnung 74 zu erzeugen.

Ein weiterer Vorteil der Gestaltung des Ankerelements 22 und des Halteelements 24 gemäß zumindest einigen Ausführungsbeispielen besteht darin, dass der Haltebereich 70 keine vordefinierte Dicke (bzw. Höhe) aufweisen muss. Die Rastverbindung 72 definiert eine Mehrzahl möglicher Relativlagen, in denen das Ankerelement 22 und das Halteelement 24 miteinander verrasten können. Mit anderen Worten erlaubt die Rastverbindung 72 ein Nachspannen der Verbindung zwischen dem Ankerelement 22 und dem Halteelement 24. Ein Vorteil ist, dass die Befestigungseinheit 20 nicht hochgenau an eine vorhandene Dicke des Gewebeabschnitts 36 angepasst werden muss. Dies erhöht die Flexibilität.

Anhand der Fig. 2 bis 5 wurde eine Ausführungsform der Rastverbindung veranschaulicht, bei der der Verbindungsdorn 60 dem Ankerelement 22 und die Rastausnehmung 46 dem Halteelement 24 zugeordnet ist. Gleichwohl ist grundsätzlich auch eine umgekehrte Zuordnung vorstellbar. Eine entsprechende beispielhafte Ausgestaltung wird nachfolgend anhand der Fig. 6 bis 9 veranschaulicht.

Ähnlich wie die Fig. 2 und 3 veranschaulichen die Fig. 6 und 7 ein Halteelement 24 und ein Ankerelement 22 einer Befestigungseinheit 20. Das Halteelement 24 ist mit einer Kontaktfläche 40 und einer Endfläche 42 versehen, die voneinander abgewandt sind, wobei die Kontaktfläche 40 distalseitig und die Endfläche 42 proximalseitig angeordnet ist. Zwischen der Kontaktfläche 40 und der Endfläche 42 erstreckt sich ein Grundkörper 44.

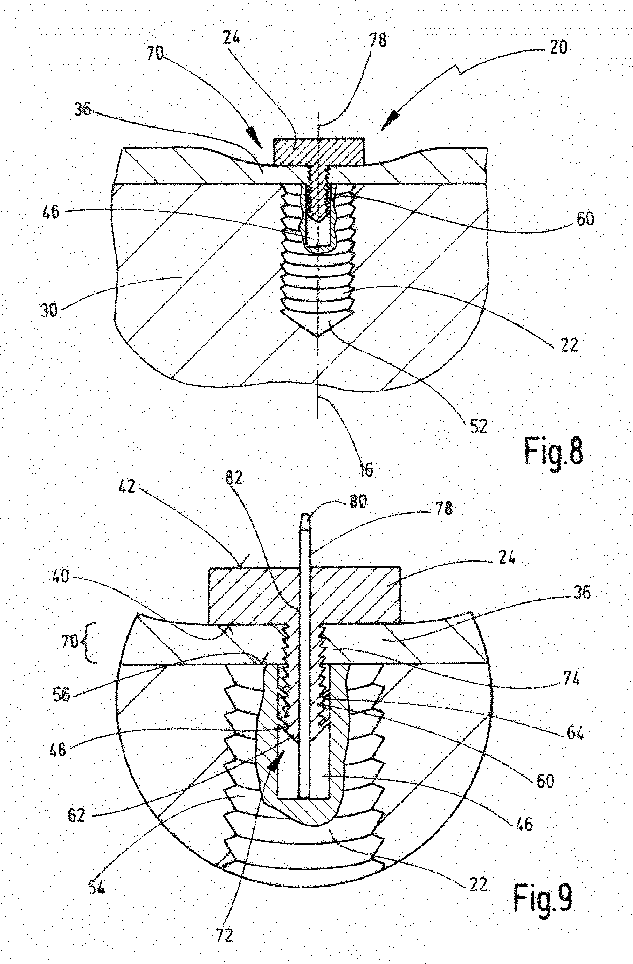

Das in Fig. 7 veranschaulichte Ankerelement 22 umfasst einen Schaftkörper 50 mit einer Spitze 52 und einer Befestigungskontur 54. Am proximalseitigen Ende des Schaftkörpers 50 ist eine Gegenfläche 56 ausgebildet, die gemeinsam mit der Kontaktfläche 40 im verrasteten Zustand des Ankerelements 22 und des Halteelements 24 einen Haltebereich 70 definiert, vergleiche auch Fig. 8 und Fig. 9.

Im Gegensatz zur anhand der Fig. 2 bis 5 veranschaulichten Ausführungsform ist bei der anhand der Fig. 6 bis 9 veranschaulichten Ausführungsform der Verbindungsdorn 60 dem Halteelement 24 zugeordnet. Demgemäß ist die Rastausnehmung 46 dem Ankerelement 22 zugeordnet.

Der Verbindungsdorn 60 erstreckt sich ausgehend von der Kontaktfläche 40 in Richtung auf das Ankerelement 22, also in Richtung auf ein distales Ende der Befestigungseinheit 20. Im Gegensatz dazu erstreckt sich der Verbindungsdorn 60 bei dem anhand der Fig. 2 bis 5 veranschaulichten Ausführungsbeispiel ausgehend von der Gegenfläche 56 in Richtung auf das Halteelement 24, also in Richtung auf ein proximales Ende der Befestigungseinheit 20. Der Verbindungsdorn 60 ist grundsätzlich ähnlich einem Kammnagel oder Ankernagel gestaltet. Die Umfangskerbung 64 kann grundsätzlich auch spiralförmig, also ähnlich einem Gewinde, ausgebildet sein.

Bei der anhand der Fig. 6 bis 9 veranschaulichten Ausgestaltung der Befestigungseinheit 20 muss beachtet werden, dass der zu befestigende Gewebeabschnitt 36 durch den Verbindungsdorn 60 von proximal nach distal, also in Richtung auf das Ankerelement 22 durchstoßen oder penetriert wird. Da jedoch der Gewebeabschnitt 36 das Ankerelement 22 zumindest abschnittsweise überdeckt, ist es unter Umständen mit Schwierigkeiten verbunden, mit dem Verbindungsdorn 60 die Rastausnehmung 46 "zu finden". Dies muss ja gewissermaßen "blind" erfolgen. Grundsätzlich kann die Spitze 62 auch als Einführhilfe dienen, um die Rastausnehmung 46 zu finden.

Gleichwohl ist es gemäß zumindest einigen Ausführungsformen vorgesehen, die Montage des Halteelements 24 zu vereinfachen, indem ein Führungsstift 78 bereitgestellt wird, der dem Ankerelement 22 zugeordnet ist. Der Führungsstift 78 ist in der Rastausnehmung 46 am Ankerelement 22 aufgenommen, verschließt diese jedoch nicht vollständig. Mit anderen Worten ist eine radiale Erstreckung bzw. ein Durchmesser des Führungsstiftes 78 deutlich kleiner als eine radiale Erstreckung bzw. ein Durchmesser der Rastausnehmung 46. In diesem Zusammenhang wird auch auf die in Fig. 9 gezeigte vergrößerte Teildarstellung der Anordnung gemäß Fig. 8 verwiesen. Der Führungsstift 78 ist beispielhaft mit einer Spitze 80 (Fig. 9) versehen. Dem Führungsstift 78 ist eine Führungsausnehmung 82 zugeordnet, die sich im Halteelement 24 erstreckt. Die Führungsausnehmung 82 ist bei dem Ausführungsbeispiel gemäß den Fig. 6 bis 9 als Durchgangsbohrung im Halteelement 24 ausgebildet, die sich durch den Grundkörper 44 und durch den Verbindungsdorn 60 erstreckt.

Der Führungsstift 78 dient als Orientierungshilfe. Nach dem Setzen des Ankerelements 22 kann der Gewebeabschnitt 36 über dem Ankerelement 22 positioniert werden. Hierbei kann der Führungsstift 78 den Gewebeabschnitt 36 durchdringen. Sodann ist der Führungsstift 78 für das Halteelement 24 "sichtbar" bzw. "spürbar". Das Halteelement 24 und der Verbindungsdorn 60 können entsprechend positioniert werden, so dass die Führungsausnehmung 82 über den Führungsstift 78 gestülpt wird. Dies beinhaltet eine Ausrichtung des Verbindungsdorns 60 sowie des Halteelements 24 in einer gewünschten Relativorientierung in Bezug auf das Ankerelement 22. Sämtliche Elemente 22, 24, 60 sind somit konzentrisch zur Längsachse 16 ausgerichtet und können miteinander gefügt werden.

Beispielhaft ist der Führungsstift 78 als separate Komponente ausgeführt und mit dem Schaftkörper 50 des Ankerelements 22 gefügt. Es ist jedoch auch vorstellbar, den Schaftkörper 50 und den Führungsstift 78 integral auszuführen, wobei die Möglichkeit besteht, Sollbruchstellen oder Ähnliches einzufügen.

Die Fig. 8 und 9 veranschaulichen einen gefügten Zustand, bei dem das Ankerelement 22 und das Halteelement 24 zwischen sich einen Haltebereich 70 definieren, in dem ein Gewebeabschnitt 36 aufgenommen ist. Ähnlich wie bei der bereits in Fig. 5 gezeigten Gestaltung ergibt sich auch in Fig. 9 eine Rastverbindung 72 zwischen dem Verbindungsdorn 60 und der Rastausnehmung 46, insbesondere zwischen der Umfangskerbung 64 und den Rastelementen 48. Hieran ändert die grundsätzlich umgekehrte Zuordnung des Verbindungsdorns 60 und der Rastausnehmung 46 nichts.

Im Haltebereich 70 weist der Gewebeabschnitt 36 eine Gewebeöffnung 74 auf, die vom Verbindungsdorn 60 durchragt wird. Die Rastelemente 48 sind derart ausgebildet und wirken derart mit der Umfangskerbung 64 zusammen, dass ein Eindringen des Verbindungsdorns 60 in die Rastausnehmung 46 ermöglicht ist, jedoch ein Ausfahren des Verbindungsdorns 60 aus der Rastausnehmung 46 behindert oder gar blockiert wird.

Die Rastverbindung 72 zur Fixierung des Gewebeabschnitts 36 am Knochen 30 kann in einfacher Weise herbeigeführt werden. Insbesondere besteht ein Vorteil darin, dass die Rastbewegung zur Fixierung der Verbindung der Zuführbewegung der beteiligten Komponenten 22, 24 gleichgerichtet ist. Dies vereinfacht die Handhabung der Befestigungseinheit 20 deutlich. Der Verzicht auf einen Faden oder ein ähnliches Hilfsmittel zur Befestigung hat den weiteren Vorteil, dass im Körperinneren nur wenig Raum für die Handhabung der Befestigungseinheit 20 erforderlich ist. Dies trägt weiter dazu bei, mögliche Traumata zu verhindern, zumindest zu minimieren.

Fig. 10 veranschaulicht anhand einer schematischen Teildarstellung in seitlicher Ansicht eine weitere beispielhafte Ausführungsform einer Befestigungseinheit 20, deren Merkmale grundsätzlich mit den zuvor bereits beschriebenen Ausgestaltungen kombinierbar sind.

Die Befestigungseinheit 20 umfasst ein Ankerelement 22 und ein Halteelement 24, die über eine Verbindung, umfassend einen Verbindungsdorn 60 sowie eine Rastausnehmung 46, miteinander verrastet oder verschnappt werden können. In der Rastausnehmung 46 sind Rastelemente 48, etwa in Form von Widerhaken ausgebildet, die mit einer Umfangskerbung 64 am Verbindungsdorn 60 zusammenwirken. Grundsätzlich ist es auch vorstellbar, Rastelemente in Form von Haken, Widerhaken oder Ähnlichem am Verbindungsdorn 60 auszubilden und demgemäß eine Umfangskerbung in der Rastausnehmung 46 auszubilden.

Am Halteelement 24 ist eine Kontaktfläche 40 ausgebildet, die mit einer Gegenfläche 56 am Ankerelement 22 zusammenwirkt, um einen Gewebeabschnitt 36 zwischen dem Ankerelement 22 und dem Halteelement 24 festzulegen, vergleiche auch die Fig. 4, 5 sowie 8, 9.

Es ist gemäß zumindest einigen Ausführungsformen vorgesehen, die Kontaktfläche 40 und/oder die Gegenfläche 56 nicht vollständig flach als Kreisfläche bzw. Ringfläche mit im Wesentlichen ebener Erstreckung auszubilden.

Stattdessen kann die Kontaktfläche 40 mit Erhebungen 86 versehen sein, die sich beispielhaft mit Senkungen 88 abwechseln. Gleichermaßen kann die Gegenfläche 56 mit Erhebungen 90 versehen sein, die sich mit Senkungen 92 abwechseln. Demgemäß kann die Kontaktfläche 40 und/oder die Gegenfläche 56 zumindest abschnittsweise wellenartig gestaltet sein. Andere Arten von Erhebungen 86, 90 bzw. Senkungen 88, 92 sind ohne weiteres denkbar. Dies kann beispielhaft domartige Erhebungen, muldenförmige Vertiefungen, Spitzen, Senkungen und Ähnliches umfassen.

Vorzugsweise sind die Konturen der Kontaktfläche 40 und der Gegenfläche 56 aneinander angepasst, so dass im gefügten, verrasteten Zustand Erhebungen 86 der Kontaktfläche 40 Senkungen 92 der Gegenfläche 56 zugeordnet sind. Gleiches gilt für Erhebungen 90 der Gegenfläche 56, die Senkungen 88 der Kontaktfläche 40 zugeordnet sind.

Durch die bewusst nicht ebene Gestaltung der Kontaktfläche 40 und der Gegenfläche 56 kann die traumatische Belastung des eingespannten Gewebeabschnitts 46 weiter reduziert werden. Insbesondere kann sich eine Vergrößerung der effektiven Kontaktfläche ergeben. Ferner ergibt sich durch die unebene Gestaltung der miteinander korrespondierenden Kontaktfläche 40 sowie Gegenfläche 56 eine Erhöhung der (seitlichen) Haltekräfte, da gewissermaßen die Reibung zwischen dem Gewebeabschnitt 36 und den angrenzenden Flächen 40, 56 durch eine entsprechende Labyrinth- oder Formschlussgestaltung erhöht wird.

Beispielhaft umfassen die Kontaktfläche 40 und die Gegenfläche 56 eine Wellkontur oder Wellenkontur, die sich radial ausgehend von der Längsachse 16 nach außen erstreckt. Mit anderen Worten erstrecken sich Wellenberge und Wellentäler sternförmig nach außen. Es ist jedoch auch eine Gestaltung denkbar, bei denen benachbarte Wellenberge und Wellentäler konzentrisch zueinander ringartig angeordnet sind. Abweichende Muster und Gestaltelemente sind selbstverständlich vorstellbar. Es ist grundsätzlich auch vorstellbar, lediglich bei der Kontaktfläche 40 oder bei der Gegenfläche 56, entsprechende Konturen mit Erhebungen 86, 90 vorzusehen.

Fig. 11 veranschaulicht eine perspektivische Ansicht einer insgesamt mit 10 bezeichneten Handhabungsvorrichtung, die ein Montagewerkzeug 12 sowie eine Befestigungseinheit 20 umfasst, die am Montagewerkzeug 12 aufgenommen ist. In diesem Zusammenhang wird auch auf die Teildarstellung in Fig. 1 verwiesen. Die Befestigungseinheit 20 ist beispielhaft gemäß den Ausführungsformen gestaltet, die in Zusammenhang mit den Fig. 2 bis 10 veranschaulicht wurden. Das Montagewerkzeug 12 umfasst ein Handstück 96, das ein proximales Ende des Montagewerkzeugs 12 definiert. Ferner ist eine Schaftbaugruppe 14 vorgesehen, die sich ausgehend vom Handstück 96 in Richtung auf ein distales Ende erstreckt. Die Schaftbaugruppe 14 umfasst einen Aufnahmeabschnitt 98, an dem die Befestigungseinheit 20 aufnehmbar ist.

Vorzugsweise ist das Montagewerkzeug 12 dazu ausgestaltet, sowohl das Ankerelement 22 als auch das Halteelement 24 zu befestigen. Dies umfasst ein Einsetzen des Ankerelements 22 in den Knochen 30, vergleiche wiederum Fig. 1. Ferner umfasst die Befestigung des Halteelements 24 ein Verrasten des Halteelements 24 mit dem zuvor befestigten Ankerelement 22. Dies erfolgt unter Einbeziehung eines Gewebeabschnitts 36.

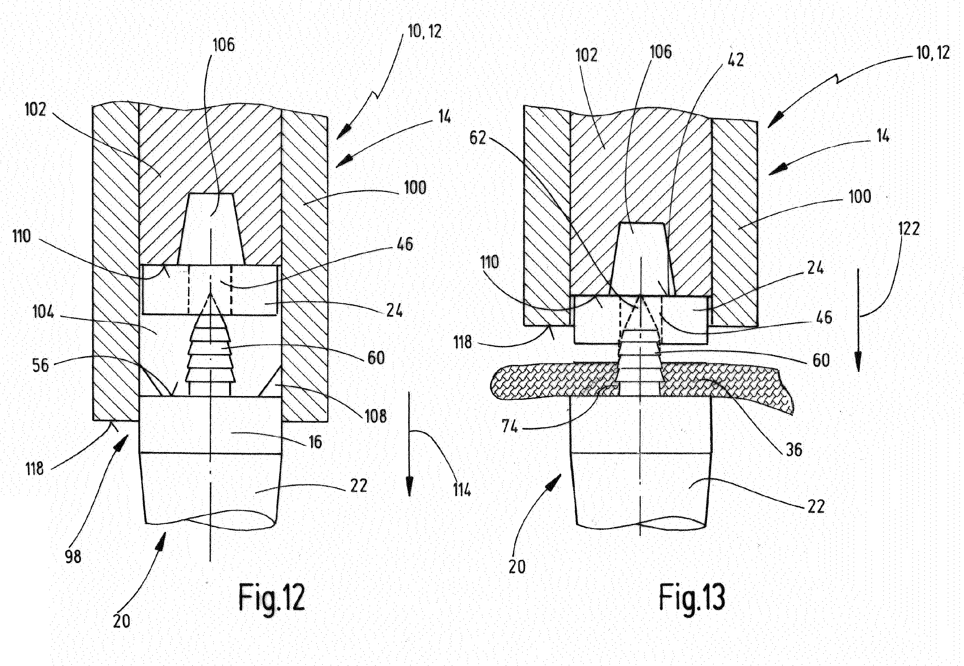

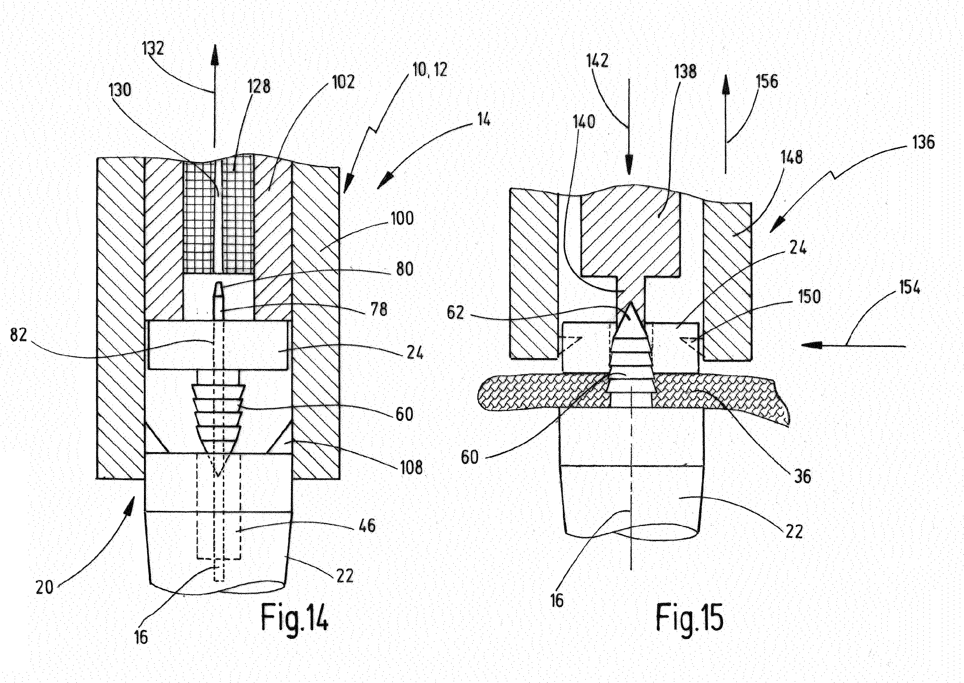

Beispielhafte Ausgestaltungen des Montagewerkzeugs 12 werden nachfolgend mit Bezugnahme auf die in den Fig. 12 und 13 gezeigte Ausführungsform sowie die anhand der Fig. 14 veranschaulichte Ausführungsform erläutert.

Fig. 12 und Fig. 13 zeigen eine geschnittene Teildarstellung des Aufnahmeabschnitts 98 der Schaftbaugruppe 14. Der jeweils gezeigten Handhabungsvorrichtung 10 ist ferner eine Befestigungseinheit 20 zugeordnet, die ein Ankerelement 22 sowie ein Halteelement 24 umfasst.

Das Ankerelement 22 sowie das Halteelement 24 sind am Aufnahmeabschnitt 98 der Schaftbaugruppe 14 aufgenommen. Fig. 12 zeigt einen Zustand des Montagewerkzeugs 12, in dem das Ankerelement 22 und das Halteelement 24 voneinander in definierter Weise beabstandet gehalten sind. In diesem Zustand können sowohl das Ankerelement 22 als auch das Halteelement 24 in Richtung auf den Knochen 30 zugeführt werden. Es ist jedoch noch nicht vorgesehen, in diesem Zustand das Ankerelement 22 und das Halteelement 24 miteinander zu verrasten. Stattdessen dient dieser Zustand des Aufnahmeabschnitts 98 vorrangig dazu, das Ankerelement 22 zu fixieren.

Die Schaftbaugruppe 14 umfasst eine erste Schaftkomponente 100 und eine zweite Schaftkomponente 102, die relativ zu einander bewegbar sind. Beispielhaft ist die erste Schaftkomponente 100 als äußere Komponente und die zweite Schaftkomponente 102 als innere Komponente ausgeführt. Die zweite Schaftkomponente 102 ist innerhalb der ersten Schaftkomponente 100 angeordnet. Das Ankerelement 22 ist an der ersten Schaftkomponente 100 aufgenommen. Das Halteelement 24 ist an der zweiten Schaftkomponente 102 aufgenommen. Die zweite Schaftkomponente 102 ist gegenüber der ersten Schaftkomponente 100 axial versetzt. Auf diese Weise ergibt sich eine Ausnehmung 104, in der das Halteelement 24 sowie der Verbindungsdorn 60 aufgenommen sind. Gleichwohl besteht in dem in Fig. 12 gezeigten Zustand noch keine Rastverbindung zwischen der Rastausnehmung 46 und dem Verbindungsdorn 60.

In der zweiten Schaftkomponente 102 ist beispielhaft eine Senkung 106 ausgebildet, in die der Verbindungsdorn 60 eintauchen kann, wenn durch eine Relativbewegung der zweiten Schaftkomponente 102 in Bezug auf die erste Schaftkomponente 100 das Halteelement 24 mit dem Ankerelement 22 verrastet wird.

An der ersten Schaftkomponente 100 sind beispielhafte Mitnehmer 108 ausgebildet, die am Ankerelement 22 angreifen. Die Mitnehmer 108 kontaktieren beispielhaft die Gegenfläche 56 am Ankerelement 22. Auf diese Weise kann durch Bewegung der Schaftbaugruppe 14 in einer Zuführrichtung 114 das Ankerelement 22 in einer Halteausnehmung 34 am Knochen 30 befestigt werden.

Es sind Ausgestaltungen denkbar, bei denen das Ankerelement 22 als Schlaganker ausgeführt ist. Demgemäß dient das Montagewerkzeug 12 vorrangig dazu, das Ankerelement 22 mit hohem Druck einzupressen. Es sind jedoch alternative Ausgestaltungen vorstellbar, bei denen das Ankerelement 22 als Schraubanker ausgeführt ist. Demgemäß ist bei diesen Ausgestaltungen das Montagewerkzeug 12 dazu ausgebildet, ein Drehmoment auf das Ankerelement 22 zu übertragen, um dieses einzuschrauben. Dies kann gleichwohl mit einer Vorschubkraft gekoppelt sein.

In den Fig. 12 und 13 ist ferner eine stirnseitige Druckfläche an der zweiten Schaftkomponente 102 mit 110 bezeichnet. Eine distale Stirnfläche der ersten Schaftkomponente 100 ist mit 118 bezeichnet. Vorzugsweise ist das Montagewerkzeug 12 mit der Schaftbaugruppe 14 derart gestaltet, dass das Ankerelement 22 derart in die Halteausnehmung 34 in einem Knochen 30 eingebracht werden kann, dass die Gegenfläche 56 bündig mit einer Oberfläche des Knochens 30 abschließt. Dies kann eine entsprechend angepasste Gestaltung des distalen Abschlusses bzw. der Stirnfläche 118 der ersten Schaftkomponente 100 beinhalten.

Fig. 13 veranschaulicht einen Zustand, in dem das Ankerelement 22 bereits am (nicht dargestellten) Knochen 30 fixiert ist. Ferner ist ein Gewebeabschnitt 36 über dem Ankerelement 22 abgelegt, wobei der Verbindungsdorn 60 mit seiner Spitze 62 den Gewebeabschnitt 36 perforiert hat, um einen Gewebeöffnung 74 auszubilden. In diesem Zustand ist die erste Schaftkomponente 100 relativ zur zweiten Schaftkomponente 102 axial in proximaler Richtung zurückversetzt. Mit anderen Worten ist die zweite Schaftkomponente 102 relativ zur ersten Schaftkomponente 100 in distaler Richtung in Richtung auf das Ankerelement 22 verlagert, vergleiche einen zugehörigen Richtungspfeil 122, der eine Montagebewegung bzw. Rastbewegung veranschaulicht.

Die zweite Schaftkomponente 102 kontaktiert mit der Anschlagfläche/Druckfläche 110 die Endfläche 42 am Halteelement 24 und drückt dieses in Richtung auf das Ankerelement 22. Dies umfasst eine Verrastung des Verbindungsdorns 60 mit der Rastausnehmung 46. Fig. 13 zeigt einen Zustand, in dem das Halteelement 24 den Gewebeabschnitt 36 noch nicht vollständig kontaktiert. Durch weiteren Druck auf das Halteelement 24 kann etwa der in den Fig. 4 und 5 gezeigte Zustand herbeigeführt werden. Hiernach kann das Montagewerkzeug 12 von der Befestigungseinheit 20 gelöst werden.

Ein Vergleich der Fig. 12 und 13 veranschaulicht, dass es von Vorteil sein kann, die Mitnehmer 108 versenkbar bzw. auslenkbar zu gestalten. Auf diese Weise kann die erste Schaftkomponente 100 das Halteelement 24 passieren, wenn das Montagewerkzeug 12 gelöst wird. Gemäß einer alternativen Ausgestaltung kann das Halteelement 24 eine radiale Erstreckung bzw. einen radialen Umfang aufweisen, die zumindest abschnittsweise kleiner als die radiale Erstreckung bzw. der Umfang des Ankerelements 22 im Bereich der Gegenfläche 56 sind. Auf diese Weise kann die erste Schaftkomponente 100 in einfacher Weise das Halteelement 24 passieren und auf das Ankerelement 22 einwirken, ohne dass es auslenkbarer Mitnehmer 108 oder dergleichen bedarf.

Fig. 14 veranschaulicht eine alternative Ausgestaltung eines Montagewerkzeugs 12, das insbesondere zur Montage der anhand der Fig. 6 bis 9 veranschaulichten Ausgestaltung der Befestigungseinheit 20 ausgestaltet ist.

Ähnlich wie bei dem anhand der Fig. 13 und 14 veranschaulichten Montagewerkzeug 12, ist eine Schaftbaugruppe 14 mit einer ersten Schaftkomponente 100 und einer zweiten Schaftkomponente 102 vorgesehen. Die erste Schaftkomponente 100 ist dem Ankerelement 22 zugeordnet. Die zweite Schaftkomponente 102 ist dem Halteelement 24 zugeordnet.

Die Schaftbaugruppe 14 ist ferner mit einer Zugeinheit 128 versehen, deren Aufgabe darin besteht, den Führungsstift 78 zu lösen und zu entfernen, wenn das Halteelement 24 in gewünschter Weise mit dem Ankerelement 22 verrastet ist.

Die Zugeinheit 128 umfasst einen Klemmabschnitt 130. Die Zugeinheit 128 ist beispielhaft innerhalb der zweiten Schaftkomponente 102 aufgenommen, die wiederum innerhalb der ersten Schaftkomponente 100 aufgenommen ist. Der Klemmabschnitt 130 kann etwa Klemmbacken oder ähnliche Elemente umfassen, die mit dem Führungsstift 48 zusammenwirken, um den Führungsstift 78 vom Ankerelement 22 zu lösen und abzuführen. Eine Zugrichtung oder Löserichtung ist in Fig. 14 mit 132 bezeichnet.

Mit besonderem Verweis auf Fig. 15 wird ein mit 136 bezeichnetes Revisionswerkzeug veranschaulicht, das ebenso der Handhabungsvorrichtung 10 zugeordnet sein kann.

Zweck des Revisionswerkzeugs 136 ist ein Lösen bzw. Entfernen des Halteelements 24 aus dem Verbund mit dem Ankerelement 22. Da sich beim Fügen des Halteelements 24 mit dem Ankerelement 22 ein Rastverbund bzw. eine Schnappverbindung ergibt, ist es von Vorteil, ein spezifisch gestaltetes Revisionswerkzeug 136 vorzusehen.

Das Revisionswerkzeug 136 kann in struktureller Hinsicht grundsätzlich dem in Fig. 11 gezeigten Montagewerkzeug 12 zumindest ähnlich gestaltet sein. Insbesondere kann ein Handstück sowie eine entsprechende Schaftbaugruppe ausgebildet sein.

Das Revisionswerkzeug 136 umfasst einen Niederhalter 138, der mit einem Druckstück 140 versehen ist. Der Niederhalter 138 ist dazu ausgebildet, auf das Ankerelement 22 einzuwirken, so dass hinreichend große Lösekräfte bzw. Abreißkräfte auf das Halteelement 24 aufgebracht werden können. Beispielhaft ist das Druckstück 140 dazu ausgestaltet, auf den Verbindungsdorn 60, insbesondere auf dessen Spitze 62 einzuwirken. In Fig. 15 veranschaulicht ein mit 142 bezeichneter Pfeil eine Halterichtung bzw. die Richtung einer aufgebrachten Haltekraft.

Ferner umfasst das Revisionswerkzeug 136 eine mit 148 bezeichnete Effektoreinheit. Die Effektoreinheit 148 umfasst zumindest einen Zughaken oder Greifer 150. Der Greifer 150 ist dazu ausgebildet, kraftschlüssig und/oder formschlüssig am Halteelement 24 anzugreifen. Eine entsprechende Wirkrichtung ist in Fig. 15 mit 154 bezeichnet. Mit anderen Worten kann der Greifer 150 vorzugsweise seitlich (radial) am Halteelement 24 angreifen bzw. das Halteelement 24 seitlich untergreifen. Auf diese Weise können Zugkräfte auf das Halteelement 24 aufgebracht werden, um das Halteelement 24 aus dem Verbund mit dem Ankerelement 22 zu lösen, vergleiche eine in Fig. 15 mit 156 bezeichnete Zugrichtung. Insgesamt kann also das Revisionswerkzeug 136 ähnlich einem Abzieher gestaltet sein.

Es ist jedoch auch vorstellbar, das Revisionswerkzeug 136 derart zu gestalten, dass die Effektoreinheit 148 bzw. der Greifer 150 dazu ausgestaltet sind, das Halteelement 24 zumindest abschnittsweise zu zerstören. Ähnlich einem Mutternsprenger kann zu diesem Zweck seitlich (Pfeil 154) auf einen Umfangsabschnitt am Halteelement 24 eingewirkt werden, um den Verbund des Halteelements 24 mit dem Ankerelement 22 zu lösen. Es versteht sich, dass auch in diesem Fall das Revisionswerkzeug 136 dazu ausgestaltet ist, das Halteelement 24 rückstandslos zu entfernen.

Wenn das Halteelement 24 entfernt ist, können erforderliche Manipulationen am Gewebeabschnitt 36 durchgeführt werden. Schlussendlich ist es möglich, mit dem Montagewerkzeug 12, vergleiche insbesondere Fig. 13, ein neues Halteelement 24 zuzuführen und am Ankerelement 22 festzulegen.

Für die Handhabung der Befestigungseinheit 20 kann ein Set umfassend ein Montagewerkzeug 12 sowie ein Revisionswerkzeug 136 für das Lösen des Halteelements 24 bereitgestellt werden.

## Patentansprüche

1. Befestigungseinheit zur fadenlosen Befestigung von Gewebe (32) an Knochen (30), wobei die Befestigungseinheit Folgendes aufweist:
- ein Ankerelement (22), das an einem Knochen (30) fixierbar ist,
- ein Halteelement (24), das mit dem Ankerelement (22) koppelbar ist, um einen Gewebeabschnitt (36) am Knochen (30) anzulagern,
wobei das Ankerelement (22) und das Halteelement (24) miteinander verrastbar sind und zwischen sich einen Haltebereich (70) ausbilden, in dem der Gewebeabschnitt (36) aufnehmbar ist,
**dadurch gekennzeichnet, dass**
am Ankerelement (22) ein Verbindungsdorn (60) ausgebildet ist, der sich zwischen einem Grundkörper (44) des Halteelements (24) und einem Schaft des Ankerelements (22) erstreckt,
dass der Verbindungsdorn (60) dazu ausgebildet ist, den Gewebeabschnitt (36) zu durchragen, wenn das Halteelement (24) und das Ankerelement (22) aufeinander zu bewegt werden,
dass der Verbindungsdorn (60) mit einer Riffelung oder Umfangskerbung (62) versehen ist, die eine Lagesicherung im verrasteten Zustand bewirkt, und
dass am Halteelement (24) eine Rastausnehmung (46) ausgebildet ist, die im verrasteten Zustand vom Verbindungsdorn (60) zumindest abschnittsweise durchragt wird.

2. Befestigungseinheit nach Anspruch 1, **dadurch gekennzeichnet, dass** das Halteelement (24) knopfartig oder tellerartig gestaltet ist und eine dem Ankerelement (22) zugewandte Kontaktfläche (40) aufweist, die dazu ausgebildet ist, im verrasteten Zustand den Gewebeabschnitt (36) in Richtung auf das Ankerelement (22) zu beaufschlagen.

3. Befestigungseinheit nach Anspruch 2, **dadurch gekennzeichnet, dass** die Kontaktfläche (40) zumindest abschnittsweise mit Erhebungen (86) versehen ist.

4. Befestigungseinheit nach Anspruch 2 oder 3, **dadurch gekennzeichnet, dass** das Ankerelement (22) eine Gegenfläche ***(56)*** aufweist, die der Kontaktfläche (40) zugewandt ist, wobei die Gegenfläche ***(56)*** und die Kontaktfläche (40) im verrasteten Zustand zwischen sich den Haltebereich (70) für den Gewebeabschnitt (36) definieren.

5. Befestiaunqseinheit nach Anspruch 4, **dadurch gekennzeichnet, dass** die Gegenfläche ***(56)*** zumindest abschnittsweise mit Erhebungen (90) versehen ist, die an Erhebungen (86) der Kontaktfläche (40) des Halteelements (24) angepasst sind, so dass sich im verrasteten Zustand ein Labyrinth oder ein Muster aus Erhebungen (86) der Kontaktfläche (40) und Erhebungen (90) der Gegenfläche ***(56)*** ergibt,

6. Befestigungseinheit nach einem der Ansprüche 1 bis 5, **gekennzeichnet durch** eine Rastverbindung (72), die eine Annäherung zwischen dem Halteelement (24) und dem Ankerelement (22) erlaubt und im verrasteten Zustand einer Lösebewegung zwischen dem Halteelement (24) und dem Ankerelement (22) entgegenwirkt.

7. Befestigungseinheit nach einem der Ansprüche 1 bis 6, **dadurch gekennzeichnet, dass** der Verbindungsdorn (60) mit einer Rastausnehmung (46) zusammenwirkt, die Rastelemente (48) aufweist.

8. Befestigungseinheit nach Anspruch 7, **dadurch gekennzeichnet, dass** die Rastelemente (48) richtungsabhängig gestaltet sind und eine Sperrrichtung aufweisen.

9. Befestigungseinheit nach einem der Ansprüche 1 bis 8, **dadurch gekennzeichnet, dass** der Verbindungsdorn (60) am Ankerelement (22) als Fortsatz in Richtung auf das Halteelement (24) ausgebildet ist.

10. Handhabungsvorrichtung zum Einbringen einer Befestigungseinheit (20), die Folgendes aufweist:
- ein Montagewerkzeug (12), und
- eine Befestigungseinheit (20) nach einem der vorhergehenden Ansprüche,
wobei das Montagewerkzeug (12) eine Schaftbaugruppe (14) mit einem distalen Aufnahmeabschnitt (98) aufweist, an dem die Befestigungseinheit (20) aufnehmbar ist,
wobei die Schaftbaugruppe (14) eine erste Schaftkomponente (100) zur Aufnahme des Ankerelements (22) und eine zweite Schaftkomponente (102) zur Aufnahme des Halteelements (24) aufweist,
wobei das Ankerelement (22) und das Halteelement (24) in einem voneinander entkoppelten Zustand axial versetzt zueinander aufnehmbar sind,
wobei die erste Schaftkomponente (100) dazu ausgebildet ist, das Ankerelement (22) dem Knochen (30) zuzuführen, und
wobei die zweite Schaftkomponente (102) dazu ausgebildet ist, das Halteelement (24) und das Ankerelement (22) miteinander zu verrasten, wenn das Ankerelement (22) fixiert ist.

11. Handhabungsvorrichtung nach Anspruch 10,
wobei die erste Schaftkomponente (100) dazu ausbildet ist, zumindest eine Vorschubkraft oder ein Drehmoment auf das Ankerelement (22) zu übertragen, und
wobei die zweite Schaftkomponente (102) relativ zur ersten Schaftkomponente (100) verschiebbar ist, um das Halteelement (24) in Richtung auf das Ankerelement (22) zu bewegen.

12. Handhabungsvorrichtung nach Anspruch 10 oder 11, ferner aufweisend eine Zugeinheit (128) die dazu ausgebildet ist, einen Führungsstift (78), der am Ankerelement (22) angeordnet ist, vom Ankerelement (22) zu lösen, wenn das Halteelement (24) mit dem Ankerelement (22) gekoppelt ist, wobei die Zugeinheit (128) vorzugsweise der Schaftbaugruppe (14) zugeordnet ist.

13. Handhabungsvorrichtung nach einem der Ansprüche 10 bis 12, ferner aufweisend ein Revisionswerkzeug (136) zum Lösen des Halteelements (24) vom Ankerelement (22), wobei das Revisionswerkzeug als Abziehwerkzeug oder als Sprengwerkzeug gestaltet ist.

## Claims

1. Fixation unit for a suture-less fixation of tissue (32) at a bone (30), wherein the fixation unit comprises the following:
- an anchor element (22) that is arranged to be fixed to a bone (30),
- a retaining element (24) that is arranged to be coupled with the anchor element (22) to attach a tissue section (36) at the bone (30),
wherein the anchor element (22) and the retaining element (24) are lockable to one another and form therebetween a retaining zone (70) in which the tissue section (36) is retainable,
**characterized in that**
at the anchor element (22) there is formed a connecting spike (60) that extends between a base body (44) of the retaining element (24) and a shaft of the anchor element (22),
**in that** the connecting spike (60) is arranged to extend through the tissue section (36) when the retaining element (24) and the anchor element (22) approach one another,
**in that** the connecting spike (60) is provided with a fluting or circumferential notching (62) that effects a positional securing in the locked state, and
**in that** at the anchor element (22) a locking recess (46) is formed, which is, in the locked state, at least sectionally traversed by the connecting spike (60).

2. Fixation unit according to claim 1, **characterized in that** the retaining element (24) is button shaped or plate shaped and provided with a contact surface (40) facing the anchor element (22), wherein the contact surface (40) is arranged to bias the tissue section (36), in the locked state, towards the anchor element (22).

3. Fixation unit according to claim 2, **characterized in that** the contact surface (40) is at least sectionally provided with elevations (86).

4. Fixation unit according to claim 2 or 3, **characterized in that** the anchor element (22) comprises a counter surface (56) that is facing the contact surface (40), wherein the counter surface (56) and the contact surface (40) define therebetween, in the locked state, the retaining zone (70) for the tissue section (36).

5. Fixation unit according to claim 4, **characterized in that** the counter surface (56) is at least sectionally provided with elevations (90) that are adapted to elevations (86) of the contact surface (40) of the retaining element (24) so that a labyrinth or a pattern of elevations (86) of the contact surface (40) and elevations (90) of the counter surface (56) is formed in the locked state.

6. Fixation unit according to any of the claims 1 to 5, **characterized by** a snap-lock connection (72) that enables an approaching between the retaining element (24) and the anchor element (22) and that counteracts, in the locked state, a detachment movement between the retaining element (24) and the anchor element (22).

7. Fixation unit according to any of the claims 1 to 6, **characterized in that** the connecting spike (60) cooperates with a locking recess (46) that comprises locking elements (48).

8. Fixation unit according to claim 7, **characterized in that** the locking elements (48) are formed to be direction dependent and comprise a blocking direction.

9. Fixation unit according to any of the claims 1 to 8, **characterized in that** the connecting spike (60) is formed at the anchor element (22) as an extension towards the retaining element (24).

10. Handling device for inserting a fixation unit (20), comprising the following:
- a mounting tool (12), and
- a fixation unit (20) in accordance with any of the preceding claims,
wherein the mounting tool (12) comprises a shaft assembly (14) having a distal support section (98) at which the fixation unit (20) is mountable,
wherein the shaft assembly (14) comprises a first shaft component (100) for taking up the anchor element (22), and a second shaft component (102) for taking up the retaining element (24),
wherein the anchor element (22) and the retaining element (24) are, in a state disengaged from one another, mountable in a state axially displaced from one another,
wherein the first shaft component (100) is arranged to feed the anchor element (22) to the bone (30), and
wherein the second shaft component (102) is arranged to lock the retaining element (24) and the anchor element (22) with one another when the anchor element (22) is fixed.

11. Handling device according to claim 10,
wherein the first shaft component (100) is arranged to transmit at least one of a feed force or a torque to the anchor element (22), and
wherein the second shaft component (102) is displaceable relative to the first shaft component (100) to move the retaining element (24) towards the anchor element (22).

12. Handling device according to claim 10 or 11, further comprising a pull unit (128) that is arranged to detach a guide pin (78) that is arranged at the anchor element (22) from the anchor element (22) when the retaining element (24) is coupled with the anchor element (22), wherein the pull unit (128) is preferably associated with the shaft assembly (14).

13. Handling device according to any of the claims 10 to 12, further comprising an inspection tool (136) for detaching the retaining element (24) from the anchor element (22), wherein the inspection tool is arranged as one of a pulling-off tool and a splitter tool.

## Revendications

1. Unité de fixation permettant de fixer sans fil un tissu (32) à un os (30), l'unité de fixation présentant les éléments suivants :
- un élément d'ancrage (22) qui peut être fixé sur un os (30),
- un élément de maintien (24) qui peut être couplé à l'élément d'ancrage (22) pour fixer une partie de tissu (36) sur l'os (30),
l'élément d'ancrage (22) et l'élément de maintien (24) pouvant être enclenchés l'un dans l'autre et réalisant ensemble une zone de maintien (70) qui peut recevoir la partie de tissu (36),
**caractérisée**
**en ce qu'**un goujon de liaison (60) qui s'étend entre un corps de base (44) de l'élément de maintien (24) et un manche de l'élément d'ancrage (22) est réalisé sur l'élément d'ancrage (22),
**en ce que** le goujon de liaison (60) est réalisé pour faire saillie à travers la partie de tissu (36) lorsque l'élément de maintien (24) et l'élément d'ancrage (22) sont rapprochés,
**en ce que** le goujon de liaison (60) est muni d'une cannelure ou d'une entaille circonférentielle (62) qui provoque un blocage de position à l'état enclenché, et
**en ce que** sur l'élément de maintien (24) est réalisé un évidement d'enclenchement (46) à travers lequel, à l'état enclenché, le goujon de liaison (60) fait saillie au moins par endroits.

2. Unité de fixation selon la revendication 1, **caractérisée en ce que** l'élément de maintien (24) est conçu à la manière d'un bouton ou d'un plateau et présente une surface de contact (40) tournée vers l'élément d'ancrage (22) et qui est réalisée, à l'état enclenché, pour solliciter la partie de tissu (36) en direction de l'élément d'ancrage (22).

3. Unité de fixation selon la revendication 2, **caractérisée en ce que** la surface de contact (40) est dotée de bossages (86) au moins par endroits.

4. Unité de fixation selon la revendication 2 ou 3, **caractérisée en ce que** l'élément d'ancrage (22) présente une surface antagoniste (56) qui est tournée vers la surface de contact (40), la surface antagoniste (56) et la surface de contact (40), à l'état enclenché, définissant ensemble la zone de maintien (70) pour la partie de tissu (36).

5. Unité de fixation selon la revendication 4, **caractérisée en ce que** la surface antagoniste (56) est dotée de bossages (90) au moins par endroits qui sont adaptés aux bossages (86) de la surface de contact (40) de l'élément de maintien (24) de sorte qu'à l'état enclenché, il en résulte un labyrinthe ou un motif de bossages (86) de la surface de contact (40) et de bossages (90) de la surface antagoniste (56).

6. Unité de fixation selon l'une quelconque des revendications 1 à 5, **caractérisée par** une liaison par enclenchement (72) qui permet un rapprochement entre l'élément de maintien (24) et l'élément d'ancrage (22) et, à l'état enclenché, agit à l'encontre d'un mouvement de libération entre l'élément de maintien (24) et l'élément d'ancrage (22).

7. Unité de fixation selon l'une quelconque des revendications 1 à 6, **caractérisée en ce que** le goujon de liaison (60) coopère avec un évidement d'enclenchement (46) qui présente les éléments d'enclenchement (48).

8. Unité de fixation selon la revendication 7, **caractérisée en ce que** les éléments d'enclenchement (48) sont conçus de manière orientée et présentent un sens de verrouillage.

9. Unité de fixation selon l'une quelconque des revendications 1 à 8, **caractérisée en ce que** le goujon de liaison (60) est réalisé sur l'élément d'ancrage (22) comme un prolongement en direction de l'élément de maintien (24).

10. Dispositif de manipulation pour introduire une unité de fixation (20), présentant les éléments suivants :
- un outil de montage (12), et
- une unité de fixation (20) selon l'une quelconque des revendications précédentes,
dans lequel l'outil de montage (12) présente un sous-ensemble formant manche (14) doté d'une partie de réception distale (98) au niveau de laquelle l'unité de fixation (20) peut être reçue,
dans lequel le sous-ensemble formant manche (14) présente un premier composant de manche (100) pour recevoir l'élément d'ancrage (22) et un deuxième composant de manche (102) pour recevoir l'élément de maintien (24),
dans lequel l'élément d'ancrage (22) et l'élément de maintien (24) peuvent être reçus dans un état découplé l'un de l'autre en étant axialement décalés l'un par rapport à l'autre,
dans lequel le premier composant de manche (100) est réalisé pour amener l'élément d'ancrage (22) jusqu'à l'os (30), et
dans lequel le deuxième composant de manche (102) est réalisé pour enclencher l'élément de maintien (24) et l'élément d'ancrage (22) l'un dans l'autre si l'élément d'ancrage (22) est fixé.

11. Dispositif de manipulation selon la revendication 10,
dans lequel le premier composant de manche (100) est réalisé pour transférer au moins une force d'avance ou un couple vers l'élément d'ancrage (22), et
dans lequel le deuxième composant de manche (102) peut être décalé par rapport au premier composant de manche (100) afin de déplacer l'élément de maintien (24) en direction de l'élément d'ancrage (22).

12. Dispositif de manipulation selon la revendication 10 ou 11, présentant en outre une unité de traction (128) qui est réalisée pour libérer de l'élément d'ancrage (22) une cheville de guidage (78) qui est disposée sur l'élément d'ancrage (22) si l'élément de maintien (24) est couplé à l'élément d'ancrage (22), l'unité de traction (128) étant de préférence associée au sous-ensemble formant manche (14).

13. Dispositif de manipulation selon l'une quelconque des revendications 10 à 12, présentant en outre un outil de révision (136) pour libérer l'élément de maintien (24) de l'élément d'ancrage (22), l'outil de révision étant conçu comme un outil d'extraction ou un outil d'éjection.
